# NEUE EUROPÄISCHE PATENTSCHRIFT

(11) **EP 1 913 073 B2**
(45) Veröffentlichungstag und Bekanntmachung des Hinweises auf die Entscheidung über den Einspruch: **14.10.2020**
(45) Hinweis auf die Patenterteilung: 04.10.2017
(21) Anmeldenummer: 06776611.3
(22) Anmeldetag: 04.08.2006
(51) Int. Cl.: C08L 5/00, C08L 33/26, C08J 3/02, A61K 8/06, A61K 8/72, A61K 9/107

(54) **ÖL-IN-WASSER EMULSIONEN**
OIL-IN-WATER EMULSIONS
EMULSIONS HUILE DANS L'EAU

(30) Priorität: 10.08.2005 DE 102005038069
(43) Veröffentlichungstag der Anmeldung: 23.04.2008
(73) Patentinhaber: Henkel AG & Co. KGaA, 40589 Düsseldorf (DE)
(72) Erfinder: HLOUCHA, Matthias, 50677 Köln (DE); STRIEPLING, Gert-Lothar, 40591 Düsseldorf (DE); DÖRING, Thomas, 41542 Dormagen (DE); WALDMANN-LAUE, Marianne, 40789 Monheim (DE); TRÄGER, Anemone, 40227 Düsseldorf (DE)
(86) Internationale Anmeldenummer: PCT/EP2006/007739
(87) Internationale Veröffentlichungsnummer: WO 2007/017196

(56) Entgegenhaltungen:
- EP-A- 1 216 695
- EP-A- 1 430 906
- EP-A- 1 535 605
- EP-A1- 1 166 770
- WO-A-99/13848
- WO-A-99/24001
- WO-A-03/099250
- WO-A1-03/099250
- WO-A1-2004/028501
- WO-A1-2004/028501
- WO-A1-2004/039338

## Beschreibung

Die vorliegende Erfindung betrifft Öl-in-Wasser-Emulsionen, enthaltend mindestens ein hydrophob modifiziertes Polysaccharid, mindestens ein Copolymer basierend auf einer Acrylamidoalkansäure, mindestens einen Fettstoff sowie Wasser und deren Verwendung in kosmetischen oder pharmazeutischen Zusammensetzungen.

Ein Problem der heute normalerweise verwendeten Öl-in-Wasser-Emulsionen (O/W-Emulsionen) für kosmetische oder pharmazeutische Anwendungen besteht darin, dass sie aufgrund der darin enthaltenen kurzkettigen Emulgatoren zu Hautreizungen, Hautrötungen oder zu trockener Haut führen können (siehe z.B. G. Imokawa, J.Soc.Cosmet.Chem., 31, 41-45 (1980)) oder aus kosmetischer Sicht nicht akzeptable sensorische Eigenschaften aufweisen.

Überraschenderweise wurde nun gefunden, dass O/W-Emulsionen, die ein hydrophob modifiziertes Polysaccharid und ein Copolymer basierend auf einer Acrylamidoalkansäure enthalten ganz besonders vorteilhafte Eigenschaften aufweisen.

Hierbei kann überraschenderweise insbesondere auf die Verwendung kurzkettiger Coemulgatoren und darüber hinaus auf die Verwendung sonstiger Tenside verzichtet und damit das Problem der Hautreizung, Hautrötung und/oder von trockener Haut vermieden werden. Die Formulierungen zeichnen sich darüber hinaus durch gute sensorische Eigenschaften aus.

Die Verwendung von hydrophob modifizierten Polysacchariden in O/W-Emulsionen ist in der Literatur bereits beschrieben, jedoch nicht in Kombination mit Copolymeren auf Basis einer Acrylalkansäure.

So beschreibt DE 10247695 Emulsionen mit substituierten Sacchariden, die frei von kurzkettigen Emulgatoren sind. Hierbei werden jedoch hohe Mengen an Polyolen eingesetzt, die nachteilige kosmetische Eigenschaften haben. Sie erhöhen zum einen die Klebrigkeit der Formulierungen und können zu Hautreizungen führen.

US 6277893 beschreibt O/W-Emulsionen, die modifizierte Polysaccharide und als zusätzlichen Emulgator Dimethicone Copolyole enthalten. In der vorliegenden Erfindung kann auf den Einsatz von Dimethicone Copolyolen verzichtet werden.

US6,699,488 beschreibt leicht abspülbare Emulsionen u.a. auf Basis von hydrophob modifizierten Polysacchariden. Eine leichte Abspühlbarkeit ist für O/W-Emulsionen im Bereich Skin-Care jedoch nicht erwünscht. Als modifizierte Polysaccharide werden des weiteren als bevorzugt solche beschrieben, die in wässriger Lösung relativ geringe Grenzflächenaktivität besitzen.

In Booten, K. and Levecke, B., "Polymeric carbohydrate-based surfactants and their use in personal care applications", SÖFW, 130, 10-16 (2004) wird die Herstellung einer leichtverteilbaren Lotion durch modifizierte Polysaccharide in Kombination mit einem modifizierten Acrylat gelehrt. Das schnelle Aufbrechen einer dünnen Lotion auf Basis von Polyacrylaten führt zu einem wässrigen Hautgefühl, dass für O/W-Skin-Care-Emulsionen nicht erwünscht ist.

EP0964054 stellt eine relevante Stoffklasse von modifizierten Polysacchariden vor, gibt aber keinen Hinweis auf die erfindungsgemäß vorteilhafte Kombination mit Copolymeren auf Basis einer Acrylalkansäure.

Ebenso sind im Stand der Technik bereits Emulsionen beschrieben, die Copolymere auf Basis einer Acrylalkansäure enthalten. Als Beispiel hierfür seien die Patentschriften EP1321129, EP1216686, EP1216695, EP1116733, US6620420, DE10029462 und WO 03/022235 genannt. Die erfindungsgemäßen vorteilhaften Ausführungsformen von Copolymeren auf Basis einer Acrylamidoalkansäure in Kombination mit hydrophob modifizierten Polysacchariden werden jedoch auch dort nicht genannt.

In der WO03/099250 A wird in Tabelle X eine O/W Emulsion offenbart, welche Natrosol^{®} Plus (Cetylhydroxyethylcellulose) und Aristoflex^{®} AVC enthält.

Die Beispiele 26-29 der WO99/13848 A offenbaren eine Zusammensetzung, enthaltend Cetylhydroxyethylcellulose und Sepigel^{®} 305.

Beispiel 2 der EP 1 166 770 A1 offenbart eine O/W-Emulsion, enthaltend Dextrinpalmitat und Hostacerin^{®} AMPS.

Das Beispiel 5 der EP 1 535 605 A1 offenbart eine O/W-Emulsion, enthaltend Aristoflex^{®} AVC und Aluminium Stärke Octenylsuccinat.

EP 1 430 906 A1 offenbart in Beispiel 3 eine O/W-Emulsion, enthaltend ein Copolymer basierend auf Ammoniumacryloyldimethyltaurat und Hydroxypropylmethylcellulose. Beispiel 9 dieser Schrift offenbart eine O/W-Emulsion, enthaltend ein Copolymer basierend auf Ammoniumacryloyldimethyltaurat und Aluminium Stärke Octenylsuccinat.

Das Beispiel 1.2 der WO99/24001 A1 offenbart eine O/W-Emulsion, enthaltend ein Copolymer basierend auf Ammoniumacryloyldimethyltaurat und Hydroxypropylmethylcellulose.

Gegenstand der vorliegenden Erfindung sind demnach O/W-Emulsionen, enthaltend folgende Bestandteile:
a) mindestens ein hydrophob modifiziertes Polysaccharid
b) 0,5 bis 5,0 Gew.-% mindestens eines Copolymers basierend auf einer Acrylamidoalkansäure,
c) mindestens einen Fettstoff,
d) Wasser,
wobei es sich bei dem Copolymer gemäß b) um ein Copolymer auf Basis einer Acrylamidoalkansäure um Polymere umfassend Einheiten der Formel -CH₂-C(R¹)(W)- und Einheiten der Formel -CH₂-C(R₂)([X]ₘ-[Y]ₙ-Z)- und/oder Einheiten der Formel -CH₂-C(R⁵)(Het)-handelt, wobei
R¹, R² und R⁵ unabhängig voneinander für Wasserstoff oder Methyl stehen,
W für CONR³R⁴ steht,
R³ für (C₁-C₆)-Alkyl-CO₂H, (C₁-C₆)-Alkyl-PO₃H oder (C₁-C₆)-Alkyl-SO3H steht,
R⁴ für Wasserstoff oder (C₁-C₆)-Alkyl steht,
X für C(O)O-, C(O)NH- oder (C₁-C₆)-Alkylen steht,
Y für (C₁-C₄)-Alkylen-oxy steht,
Z für (C₅-C₅₀)-Alkyl steht
m für 0 oder 1 steht,
Het für einen N-Heterocyclus mit 5 oder 6 Ringgliedern und 1 oder 2 Heteroatomen steht, wobei mindestens 1 Heteroatom N ist und das andere Heteroatom ausgewählt ist aus N, O oder S, und über das mindestens eine N-Heteroatom des Heterocyclus vorzugsweise Bindung an das C-Atom des Rückgrats des Polymers erfolgt, und wobei im Heterocyclus vorzugsweise mindestens eine CO-Gruppe enthalten ist, die vorzugsweise in ortho-Stellung zu dem mindestens einen N-Heteroatom angeordnet ist, und wobei der Heterocyclus gegenbenenfalls auch weitere Substituenten, insbesondere ausgewählt aus C1-6-Alkyl und Halogen, tragen kann, und wobei die Kohlenwasserstoffreste gegebenenfalls auch einoder mehrfach substituiert sein können, insbesondere durch Reste ausgewählt aus Halogen, Hydroxy, Alkoxy, Amino, Alkylamino, Dialkylamino, Aryl, Arylalkyl und cycloaliphatischen Resten,
wobei es sich bei dem hydrophob modifizierten Polysaccharid um Inulin Lauryl Carbamat oder Cetyl Hydroxyethylcellulose oder Mischungen davon handelt und es sich um eine emulgatorfreie Zusammensetzung handelt.

Ein weiterer Gegenstand der vorliegenden Erfindung ist eine O/W-Emulsion, enthaltend folgende Bestandteile:
a) mindestens ein hydrophob modifiziertes Polysaccharid
b) 0,5 bis 5,0 Gew.-% mindestens eines Copolymers basierend auf einer Acrylamidoalkansäure,
c) mindestens einen Fettstoff,
d) Wasser,
wobei es sich bei dem Copolymer gemäß b) um ein Copolymer auf Basis einer Acrylamidoalkansäure um Polymere umfassend Einheiten der Formel -CH₂-C(R¹)(W)- und Einheiten der Formel -CH₂-C(R₂)([X]ₘ-[Y]ₙ-Z)- und/oder Einheiten der Formel -CH₂-C(R⁵)(Het)-handelt, wobei
R¹, R² und R⁵ unabhängig voneinander für Wasserstoff oder Methyl stehen,
W für CONR³R⁴ steht,
R³ für (C₁-C₆)-Alkyl-CO₂H, (C₁-C₆)-Alkyl-PO₃H oder (C₁-C₆)-Alkyl-SO3H steht,
R4 für Wasserstoff oder (C₁-C₆)-Alkyl steht,
X für C(O)O-, C(O)NH- oder (C₁-C₆)-Alkylen steht,
Y für (C₁-C₄)-Alkylen-oxy steht,
Z für (C₅-C₅₀)-Alkyl steht
m für 0 oder 1 steht,
Het für einen N-Heterocyclus mit 5 oder 6 Ringgliedern und 1 oder 2 Heteroatomen steht, wobei mindestens 1 Heteroatom N ist und das andere Heteroatom ausgewählt ist aus N, O oder S, und über das mindestens eine N-Heteroatom des Heterocyclus vorzugsweise Bindung an das C-Atom des Rückgrats des Polymers erfolgt, und wobei im Heterocyclus vorzugsweise mindestens eine CO-Gruppe enthalten ist, die vorzugsweise in ortho-Stellung zu dem mindestens einen N-Heteroatom angeordnet ist, und wobei der Heterocyclus gegenbenenfalls auch weitere Substituenten, insbesondere ausgewählt aus C1-6-Alkyl und Halogen, tragen kann, und wobei die Kohlenwasserstoffreste gegebenenfalls auch einoder mehrfach substituiert sein können, insbesondere durch Reste ausgewählt aus Halogen, Hydroxy, Alkoxy, Amino, Alkylamino, Dialkylamino, Aryl, Arylalkyl und cycloaliphatischen Resten,
wobei es sich bei dem hydrophob modifizierten Polysaccharid um Inulin Lauryl Carbamat handelt.

Erfindungsgemäß ist die erfindungsgemäße Zusammensetzung frei von normalerweise eingesetzten kurzkettigen Tensiden, Emulgatoren und Coemulgatoren, insbesondere frei von nicht Silikon-haltigen Tensiden, Emulgatoren und Coemulgatoren, vor allem frei von solchen kurzkettigen Tensiden, Emulgatoren und Coemulgatoren, die ein Molekulargewicht kleiner gleich 1000 g/mol besitzen bzw. sind diese nur in sehr geringen Mengen, insbesondere zu weniger als 1,0, vorzugsweise zu weniger als 0,5, vor allem zu weniger als 0,2, insbesondere zu weniger als 0,1 Gew.-%, enthalten. Die Zusammensetzung ist hierbei frei von kurzkettigen kationischen und anionischen Tensiden, besonders bevorzugt jedoch frei von jeder Art von kurzkettigen Tensiden, also von kationischen, anionischen, amphoteren und nichtionischen Tensiden. Zusammenfassend enthält die Zusammensetzung erfuindungsgemäß keine Emulgatoren mit einem Molekulargewicht kleiner gleich 5000 g/mol und ist diesem Sinne gemäß Definition der Gesellschaft für Dermopharmazie als emulgatorfrei anzusehen.

Besondere Vorteile der erfindungsgemäßen Zusammensetzung sind, dass sie hinsichtlich der Probleme Hautreizung (Hautirritation), Hautrötung oder trockene Haut ein besseres Verhalten zeigen als die herkömmlicherweise verwendeten Zubereitungen und dass vorzugsweise diese Probleme vollständig vermieden werden können. Die erfindungsgemäßen Zusammensetzungen sind daher besonders gut geeignet zur Verwendung bei sensitiver, empfindlicher oder trockener Haut bzw. in Bereichen, in denen es leicht zu Irritationen kommen kann, beispielsweise im Bereich der Augen.

Gegenstand der vorliegenden Erfindung ist daher auch die Verwendung erfindungsgemäßer O/W-Emulsionen zur Behandlung der Gesichtshaut und/oder zur Herstellung einer Zusammensetzung zur Behandlung oder Vermeidung von empfindlicher oder trockener Haut und/oder zur Herstellung einer Zusammensetzung zur Vermeidung von Hautrötungen und Hautirritationen.
Gegenstand der vorliegenden Erfindung ist hierbei insbesondere die Verwendung erfindungsgemäßer O/W-Emulsionen zur Behandlung von Haut in empfindlichen oder sensitiven Bereichen, insbesondere im Bereich der Augen.

Gegenstand der vorliegenden Erfindung ist des weiteren die Verwendung erfindungsgemäßer O/W-Emulsionen zur Behandlung reifer Haut, zur Herstellung einer Zusammensetzung zur Behandlung gestresster, insbesondere UV-gestresster, Haut und/oder zur Herstellung einer Zusammensetzung zur Behandlung der Haut nach einer chemischen oder mechanischen Peeling-Behandlung.

Gegenstand der vorliegenden Erfindung ist ferner die Verwendung erfindungsgemäßer O/W-Emulsionen zur Herstellung einer Zusammensetzung zum Transport von Wirkstoffen in die Haut.

Weitere Vorteile der erfindungsgemäßen Zusammensetzungen sind hohe Stabilität der Zusammensetzungen gegen Phasenseparation bei verschiedenen Lagerbedingungen, ein gutes Hautgefühl, was insbesondere auf die gute Verteilbarkeit, das gute Gleitverhalten, auf fehlende Klebrigkeit, das Ausbleiben von Rückständen und das gute Einziehverhalten zurückzuführen ist, sowie gute Anwendungseigenschaften wie das Fehlen des Weißeleffekts (beim Verreiben tritt keine vorübergehende Weißfärbung auf, die auf Schaumbildung zurückzuführen ist).

Des weiteren ist vorteilhaft herauszustellen, daß die Herstellung im Kalt-Kalt-Verfahren erfolgen kann, was zum einen eine sehr energiesparende und damit kostengünstige und umweltfreundliche Herstellungsweise darstellt und des weiteren die Einarbeitung und Stabilisierung wärmeempfindlicher Wirkstoffe ermöglicht.

In einer erfindungsgemäß bevorzugten Ausführungsform beträgt der Anteil an hydrophob modifiziertem Polysaccharid von 0,1 - 3,0 Gew.-%, der Anteil an Copolymer basierend auf einer Acrylamidoalkansulfonsäure 0,1 - 3,0 Gew.-% und der Anteil an Fettstoffen 5,0 - 30,0 Gew.-%.

Der pH-Wert der erfindungsgemäßen Zusammensetzungen beträgt vorzugsweise von 5,0 bis 7,0, besonders bevorzugt von 5,3 bis 6,0.

Die erfindungsgemäßen Zusammensetzungen besitzen bei einer Temperatur von 25°C vorzugsweise eine Viskosität von 500 bis 10000 Pas bei einer Scherrate von 0,1 s⁻¹ und eine Viskosität von 1 bis 10 Pas bei einer Scherrate von 100 s⁻¹ (Methode: Rotationsrheometer, Bestimmung der Fließkurve (AR 1000-N, Fa. TA-Instruments)).

Bei der kosmetischen oder pharmazeutischen Zusammensetzung kann es sich insbesondere um eine Emulsion, eine Lotion, ein Spray, eine Creme oder ein Salbe handeln. In einer bevorzugten Ausführungsform handelt es sich um eine Zusammensetzung zur Anwendung in empfindlichen, leicht irritierbaren Bereichen, insbesondere im Bereich der Augen oder im Intimbereich oder in anderen Bereichen der Schleimhaut. Es kann sich entsprechend beispielsweise um eine Augensalbe, Augencreme oder um eine Intimsalbe handeln.

Als Applikationsort kommt entsprechend die Haut jedes Körperbereichs in Frage, insbesondere jedoch die Gesichtshaut, besonders im Bereich der Augen, oder die Schleimhaut, insbesondere im Intimbereich.

### Hydrophob modifizierte Polysaccharide

Bei dem Grundgerüst des hydrophob modifizierten Polysaccharids handelt es sich erfindungsgemäß vorzugsweise um ein wasserlösliches Polysaccharid und/oder um dessen Ether mit kurzkettigen Alkoholen. Das wasserlösliche Polysaccharid kann jede beliebige Struktur besitzen. So kann es beispielsweise linear, verzweigt, kammartig und/oder sternförmig sein, wobei jedoch lineare Polysaccharide bevorzugt sind.
Bei dem Grundgerüst des hydrophob modifizierten Polysaccharids kann es sich ferner auch um ein Copolymer oder um ein Block-Copolymer unterschiedlicher Monosaccharid-Einheiten und/oder auf unterschiedliche Weise miteinander verknüpfter Monosaccharid-Einheiten handeln.

Erfindungsgemäß verwendbare Polysaccharid-Grundgerüste sind beispielsweise ausgewählt aus der Gruppe bestehend aus Polyglucose, insbesondere Cellulose, Amylose, Amylopektin oder Dextrine, und Polyfructose sowie Hydroxyalkyl-modifizierte Derivate dieser Verbindungen. Erfindungsgemäß besonders bevorzugte Polysaccharid-Grundgerüste sind Hydroxyethylcellulose und lineare Polyfructose, insbesondere Inulin.

Das Polysaccharid, insbesondere das Inulin, umfasst in einer bevorzugten Ausführungsform im Mittel von 3 bis 100, besonders bevorzugt von 5 bis 75, vor allem von 10 bis 40, Monosaccharid-Einheiten.

Die hydrophobe Modifizierung wird vorzugsweise erreicht durch C₃₋₂₂-Alkylgruppen, insbesondere durch C₈₋₂₀-Alkylgruppen, besonders bevorzugt durch C₁₀₋₁₆-Alkylgruppen. Die Alkylgruppen können linear oder verzweigt, gesättigt oder ungesättigt sein, sind jedoch vorzugsweise linear und ungesättigt.
Die hydrophoben Gruppen sind vorzugsweise über eine Alkyl-Etherbindung oder über eine Alkyl-Urethanbindung vorzugsweise an die Hydroxygruppen des Polysaccharids gebunden. Die hydrophoben Gruppen können aber auch über einen Linker an das Polysaccharid-Grundgerüst gebunden sein, beispielsweise über einen Polyether-Linker, insbesondere einen Polyethylenglykol-Linker.

Der Modifizierungsgrad des hydrophob modifizierten Polysaccharids, d.h. der Quotient aus Anzahl an modifizierenden Gruppen und Anzahl an Monomer-Einheiten, beträgt vorzugsweise von 0,1 bis 2,0, besonders bevorzugt von von 0,2 bis 1,0 vor allem von 0,4 bis 0,8.

Die hydrophoben Gruppen sowie unabhängig davon das Polysaccharid-Rückgrat können gegebenenfalls auch ein- oder mehrfach substituiert sein, insbesondere durch Reste ausgewählt aus Halogen, insbsondere Fluor, Chlor oder Brom, Hydroxy, Alkoxy, insbesondere C₁₋₆-Alkoxy, Amino, Alkylamino, insbesondere C₁₋₆-Alkylamino, Aryl, insbesondere C₆₋₁₀-Aryl, Arylalkyl, insbesondere C₆₋₁₀-Aryl-C₁₋₆-alkyl, Carboxy, Carboxyester, insbesondere Carboxy-C₁₋₆-alkylester, und cycloaliphatischen Resten.

Erfindungsgemäß eingesetzte hydrophob modifizierte Polysaccharide sind grenzflächenaktiv und senken die Grenzflächenspannung von Wasser gegenüber Luft und von Wasser gegenüber Öl deutlich ab. Bevorzugt ist hierbei eine Absenkung der Oberflächenspannung einer 1%igen Lösung bei auf kleiner als 60 mN/m und die Absenkung der Grenzflächenspannung einer 1%igen Lösung bei gegenüber kosmetischen organischen Ölen, insbesondere gegenüber Decyl Oleate, auf Werte unter 25, vorzugsweise unter 20 mN/m (gemessen bei 25°C nach folgenden Methoden: Ring-Tensio-Meter (Fa. Lauda) und Drop-Shape-Analysis (Fa. Krüss)).

Erfindungsgemäß bevorzugt eingesetzte hydrophob modifizierte Polysaccharide sind offenbart in EP 964054 A1 sowie in Biomacromolecules (2001, Band 2, S. 1256-1259).

Erfindungsgemäß besonders bevorzugt eingesetzte hydrophob modifizierte Polysaccharide sind Polysaccharid-N-alkylurethane der Firma Orafti, vor allem Inulin Lauryl Carbamate (Inutec SP-1, Fa. Orafti), sowie hydrophob modifizierte Hydroxyethylcellulose der Firma Hercules, insbesondere Cetyl Hydroxyethylcellulose (Natrosol Plus CS 330, Polysurf, Fa. Hercules).

Die Menge an hydrophob modifizierten Polysacchariden in den erfindungsgemäßen Zusammensetzungen beträgt vorzugsweise zwischen 0,1 und 5,0, besonders bevorzugt zwischen 0,2 und 3,0 Gew.-%.

### Copolymere auf Basis einer Acrylamidoalkansäure

Bei der Acrylamidoalkansäure-Einheit, die als Monomer zur Herstellung des Copolymers verwendet werden kann, handelt es sich in einer bevorzugten Ausführungsform um eine Acrylamido- oder Methacrylamidoethan-, -propan-, -butan-, -pentan- oder hexancarbon-, sulfon- oder phosphonsäure, wobei der Alkylrest verzweigt oder unverzweigt sein kann. Besonders bevorzugt handelt es sich um 2-Acrylamido-2-Methyl-Ethan-, -Propan- oder -Butansulfonsäure, wobei 2-Acrylamido-2-Methyl-Propansulfonsäure ganz besonders bevorzugt ist.

Als weiteres Monomer zur Herstellung des Copolymers wird in einer bevorzugten Ausführungsform neben der Acrylamidoalkansäure-Einheit eine mittels Alkylpolyether-Gruppen und/oder Alkylalkohol-Gruppen veresterte Acryl- oder Methacrylsäure eingesetzt. Die Acryl- oder Methacrylsäure ist hierbei vorzugsweise mit einer Polalkylenglykolgruppe einer Länge von zwischen 5 und 50 Einheiten, besonders bevorzugt von zwischen 10 und 40 Einheiten, vor allem von zwischen 20 und 30 Einheiten, insbesondere von etwa 25 Einheiten, verestert, an die sich vorzugsweise ein Alkylrest einer Länge C₁₀₋₃₅, besonders bevorzugt C₁₆₋₂₈, vor C₂₀₋₂₄, insbesondere C₂₂ anschließt, wobei der Alkylrest verzweigt oder unverzweigt sein kann, unverzweigte Reste jedoch bevorzugt sind, und wobei die Polyalkylengruppe vorzugsweise ausgewählt ist aus Polyethylenglykol oder Polypropylenglykol oder beliebigen Mischungen davon, wobei eine reine Polyethylenglykolgruppe bevorzugt ist. Alternativ zu einer Carbonsäureesterbindung kann auch eine Carbonsäureamidbindung ausgebildet sein oder eine Bindung über eine (C₁-C₆)-Alkylen-Gruppe, insbesondere über eine Methylen-Gruppe, vorliegen.

In einer weiteren bevorzugten Ausführungsform wird als weiteres Monomer zur Herstellung des Copolymers neben der Acrylamidoalkansäure-Einheit ein Vinyl-Heterocyclus eingesetzt, wobei es sich bei dem Heterozyklus vorzugsweise um einen N-Heterocyclus mit 5 oder 6 Ringgliedern und 1 oder 2 Heteroatomen handelt, wobei mindestens ein Heteroatom N ist und das andere Heteroatom ausgewählt ist aus N, O oder S, und wobei vorzugsweise über das mindestens eine N-Heteroatom des Heterocyclus Bindung an die Vinyl-Gruppe erfolgt, und wobei im Heterocyclus vorzugsweise mindestens eine CO-Gruppe enthalten ist, die vorzugsweise in ortho-Stellung zu dem mindestens einen N-Heteroatom angeordnet ist, und wobei der Heterocyclus gegenbenenfalls auch weitere Substituenten, insbesondere ausgewählt aus C₁₋₆-Alykl und Halogen, tragen kann.

Bei dem erfindungsgemäßen Copolymer handelt es sich um ein Polymer, das Einheiten mit der Struktur -CH₂-C(R¹)(W)- sowie Einheiten mit der Struktur -CH₂-C(R²)([X]ₘ-[Y]ₙ-Z)- und/oder Einheiten mit der Struktur -CH₂-C(R⁵)(Het)- umfasst, wobei
R¹, R² und R⁵ unabhängig voneinander für Wasserstoff oder Methyl stehen,
W für CONR³R⁴ steht,
R³ für (C₁-C₆)-Alkyl-CO₂H, (C₁-C₆)-Alkyl-PO₃H oder (C₁-C₆)-Alkyl-SO₃H steht, wobei (C₁-C₆)-Alkyl-SO₃H bevorzugt ist,
R⁴ für Wasserstoff oder (C₁-C₆)-Alkyl steht,
X für C(O)O-, C(O)NH- oder (C₁-C₆)-Alkylen, insbesondere Methylen, steht,
Y für (C₁-C₄)-Alkylen-oxy, insbesondere Ethylenoxy und/oder Propylenoxy, steht,
Z für (C₅-C₅₀)-Alkyl steht
m für 0 oder 1 steht,
Het für einen einen N-Heterocyclus mit 5 oder 6 Ringgliedern und 1 oder 2 Heteroatomen steht, wobei mindestens 1 Heteroatom N ist und das andere Heteroatom ausgewählt ist aus N, O oder S, und über das mindestens eine N-Heteroatom des Heterocyclus vorzugsweise Bindung an das C-Atom des Rückgrats des Polymers erfolgt, und wobei im Heterocyclus vorzugsweise mindestens eine CO-Gruppe enthalten ist, die vorzugsweise in ortho-Stellung zu dem mindestens einen N-Heteroatom angeordnet ist, und wobei der Heterocyclus gegenbenenfalls auch weitere Substituenten, insbesondere ausgewählt aus C₁₋₆-Alykl und Halogen, tragen kann.

Alle der zuvor genannten Kohlenwasserstoffreste können gegebenenfalls auch ein- oder mehrfach substituiert sein, insbesondere durch Reste ausgewählt aus Halogen, insbsondere Fluor, Chlor oder Brom, Hydroxy, Alkoxy, insbesondere C₁₋₆-Alkoxy, Amino, Alkylamino, insbesondere C₁₋₆-Alkylamino, Dialkylamino, insbesondere Di-C₁₋₆-Alkylamino, Aryl, insbesondere C₆₋₁₀-Aryl, Arylalkyl, insbesondere C₆₋₁₀-Aryl-C₁₋₆-alkyl und cycloaliphatischen Resten.

Es können hierbei auch unterschiedliche Ausführungsformen der zuvor genannten Einheiten im Molekül enthalten sein, die unterschiedliche Reste und/oder Substituenten aufweisen.

Die Polymere haben vorzugsweise die Eigenschaft, dass ihre 0,5%igen wäßrigen Lösungen eine Fließgrenze von größer gleich 0,1 Pa, besonders bevorzugt größer gleich 0,5 Pa erreichen und deren 2%ige wäßrige Lösungen eine Fließgrenze von größer gleich 10 Pa, besonders bevorzugt größer gleich 40 Pa aufweisen.

Der Anteil an Acrylamidoalkansäure-Einheiten am Gesamtpolymer liegt in einer bevorzugten Ausführungsform über 90 %, insbesondere zwischen 95 und 99,5 %.

Als erfindungsgemäß besonders bevorzugte Copolymere seien Mischcopolymere aus Acryloyldimethyltaurat und Vinylpyrrolidon genannt, die unter den Handelsnamen Aristoflex AVC (Clariant) im Handel erhältlich sind. Mischcopolymere aus Acryloyldimethyltaurat und Beheneth-25 Methacrylat (Aristoflex HMB (Clariant)) sind erfindungsgemäß ebenfalls verwendbar, aber weniger bevorzugt.

Die Menge an Copolymer auf Basis einer Acrylamidoalkansäure in den erfindungsgemäßen Zusammensetzungen beträgt vorzugsweise zwischen 0,1 und 3,0, besonders bevorzugt zwischen 0,5 und 2,5 Gew.-%.

### Fettstoffe

Die kosmetischen oder pharmazeutischen Zusammensetzungen enthalten weiterhin Fettstoffe. Unter Fettstoffen sind Fettsäuren, Fettalkohole, natürliche und synthetische kosmetische Ölkomponenten sowie natürliche und synthetische Wachse zu verstehen, die sowohl in fester Form als auch flüssig in wässriger oder öliger Dispersion vorliegen können.

Bei dem Fettstoff kann es sich insbesondere um ein unpolares oder polares flüssiges Öl, das natürlich oder synthetisch sein kann, handeln. Die Ölkomponente kann insbesondere ausgewählt sein aus
- pflanzlichen Ölen, insbesondere Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssigen Paraffinölen, Isoparaffinölen , z. B. Isohexadecan und Isoeicosan, aus hydrogenierten Polyalkenen, insbesondere Poly-1-decenen (im Handel erhätlich als Nexbase 2004, 2006 oder 2008 FG (Fortum, Belgien)), aus synthetischen Kohlenwasserstoffen, z.B. 1,3-Di-(2-ethyl-hexyl)-cyclohexan (Cetiol® S), sowie aus flüchtigen und nichtflüchtigen Siliconölen, die cyclisch, wie z. B. Decamethylcyclopentasiloxan und Dodecamethylcyclohexasiloxan, oder linear sein können, z. B. lineares Dimethylpolysiloxan, im Handel erhältlich z. B. unter der Bezeichnung Dow Corning^{®} 190, 200, 244, 245, 344, 345 oder 350 und Baysilon^{®} 350 M,
- Di-n-alkylethern mit insgesamt zwischen 12 bis 36 C-Atomen, insbesondere 12 bis 24 C-Atomen, wie beispielsweise Di-n-octylether (im Handel erhältlich als Cetiol® OE), Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether.
- Esterölen. Unter Esterölen sind zu verstehen die Ester von C₆-C₃₀-, insbesondere C₆-C₂₂-Fettsäuren, mit C₂-C₃₀-, insbesondere C₂-C₂₄-Fettalkoholen. Beispiele für eingesetzte Fettsäurenanteile in den Estern sind Capronsäure, Caprylsäure, 2-Ethyl-hexansäure, Caprinsäure, Laurinsäure, Isotridecansäure, My-ri-stinsäure, Palmitinsäure, Palmitoleinsäure, Stearinsäure, Isostearinsäure, Ölsäure, Elaidinsäure, Petroselinsäure, Linolsäure, Linolensäure, Elaeo-stearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosyn-these oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassi-dylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß einsetzubar sind insbesondere Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat (Cetiol®V), Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Olsäuredecylester (Cetiol® V),
- Dicarbonsäureestern wie Di-n-butyladipat, Di-(2-ethylhexyl)-adipat, Di-(2-ethylhexyl)-succinat und Düsotridecylacelaat sowie Diolester wie Ethylenglykol-dioleat, Ethylenglykoldüsotridecanoat, Propylenglykol-di(2-ethylhexanoat), Propylenglykoldiisostearat, Propylenglykoldipelargonat, Butandioldiisostearat, Neopentylglykoldicaprylat,
- symmetrischen, unsymmetrischen oder cyclischen Estern der Kohlensäure mit Fettalkoholen, beispielsweise beschrieben in der DE-OS 197 56 454, Glycerincarbonat oder Dicaprylylcarbonat (Cetiol® CC),
- Mono-, Di- und Trifettsäureestern, insbesondere Trifettsäureestern, von gesättigten und/oder ungesättigten linearen und/oder verzweigten Fettsäuren, insbesondere C₆-C₂₂-Fettsäuren, mit Glycerin, wie beispielsweise Triglyceride der Caprinsäure und/oder Caprylsäure, Monomuls® 90-018, Monomuls® 90-L12 oder Cutina® MD.

Die polare Ölkomponente kann weiterhin ausgewählt sein aus verzweigten Alkanolen, z. B. Guerbet-Alkoholen mit einer einzigen Verzweigung am Kohlenstoffatom 2 wie 2-Hexyldecanol, 2-Octyldodecanol, Isotridecanol und Isohexadecanol, aus Alkandiolen, z. B. den aus Epoxyalkanen mit 12 - 24 C-Atomen durch Ringöffnung mit Wasser erhältlichen vicinalen Diolen, aus Etheralkoholen, z. B. den Monoalkylethern des Glycerins, des Ethylenglycols, des 1,2-Propylenglycols oder des 1,2-Butandiols, aus Dialkylethern mit jeweils 12 - 24 C-Atomen, z. B. den Alkyl-methylethern oder Di-n-alkylethern mit jeweils insgesamt 12 - 24 C-Atomen, insbesondere Di-n-octylether (Cetiol^{®}OE ex Cognis), sowie aus Anlagerungsprodukten von Ethylenoxid und/oder Propylenoxid an ein- oder mehrwertige C₃₋₂₀-Alkanole wie Butanol und Glycerin, z. B. PPG-3-Myristylether (Witconol^{®} APM), PPG-14-Butylether (Ucon Fluid^{®} AP), PPG-15-Stearylether (Arlamol^{®} E), PPG-9-Butylether (Breox^{®} B25) und PPG-10-Butandiol (Macol^{®} 57).

Als Fettsäuren können eingesetzt werden lineare und/oder verzweigte, gesättigte und/ oder ungesättigte C₈₋₃₀-Fettsäuren. Bevorzugt sind C₁₀₋₂₂-Fettsäuren. Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Die eingesetzten Fettsäuren können eine oder mehrere Hydroxygruppen tragen. Bevorzugte Beispiele hierfür sind die α-Hydroxy-C₈-C₁₈-Carbonsäuren sowie 12-Hydroxystearinsäure.

Als Fettalkohole können eingesetzt werden gesättigte, ein- oder mehrfach ungesättigte, verzweigte oder unverzweigte Fettalkohole mit 6 - 30, bevorzugt 10 - 22 und ganz besonders bevorzugt 12 - 22 Kohlenstoffatomen. Einsetzbar im Sinne der Erfindung sind z.B. Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole.

Als natürliche oder synthetische Wachse können erfindungsgemäß eingesetzt werden feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.

Weiterhin geeignet sind die Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs^{®}) oder Polyolen mit 2 - 6 C-Atomen, Fettsäuremonoalkanolamide mit einem C₁₂₋₂₂-Acylrest und einem C₂₋₄-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/ oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen.

Besonders bevorzugt ist, die Wachskomponenten zu wählen aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen, sofern die Wachskomponente oder die Gesamtheit der Wachskomponenten bei Raumtemperatur fest sind. Insbesondere vorteilhaft können die Wachskomponenten aus der Gruppe der C₁₆₋₃₆-Alkylstearate, der C₁₀₋₄₀-Alkylstearate, der C₂₋₄₀-Alkylisostearate, der C₂₀₋₄₀-Dialkylester von Dimersäuren, der C₁₈₋₃₈-Alkylhydroxystearoylstearate, der C₂₀₋₄₀-Alkylerucate gewählt werden, ferner sind C₃₀₋₅₀-Alkylbienenwachs sowie Cetearylbehenat einsetzbar. Auch Silikonwachse, zum Beispiel Stearyltrimethylsilan/Stearylalkohol sind gegebenenfalls vorteilhaft. Besonders bevorzugte Wachskomponenten sind die Ester aus gesättigten, einwertigen C₂₀-C₆₀-Alkoholen und gesättigten C₈-C₃₀-Monocarbonsäuren, insbesondere ein C₂₀-C₄₀-Alkylstearat bevorzugt, das unter dem Namen Kesterwachs^{®} K82H von der Firma Koster Keunen Inc. erhältlich ist. Das Wachs oder die Wachskomponenten sollten bei 25° C fest sein, jedoch im Bereich von 35 - 95°C schmelzen, wobei ein Bereich von 45 - 85 °C bevorzugt ist.
Natürliche, chemisch modifizierte und synthetische Wachse können alleine oder in Kombination eingesetzt werden.

Erfindungsgemäß ist jede beliebige Kombination der oben genannten Fettstoffe einsetzbar.

In einer erfindungsgemäß bevorzugten Ausführungsform umfasst der erfindungsgemäß eingesetzte Fettstoff mindestens eines der oben genannten unpolaren oder polaren Öle.

Bevorzugte Öle sind hierbei Esteröle, Fettsäureester des Glycerins, Silikonöle sowie Mischungen davon. Ganz besonders bevorzugte Öle sind Decyloleat (Decyl Oleate) (Cetiol V), Pentaerythrityl Distearate (Cutina PES), Kokosfettalkohol-caprinat/-caprylat (Coco Caprylate/Caprate), Cetearyl Isononanoate (Cetiol SN), Jojobaester (Floraester 60, Floraester 70), Dicaprylylether (Cetiol OE), Butyrospermum Parkii (Shea Butter) (Cetiol SB 45), Prunus Amygdalus Dulcis (Sweet Almond) Oil, Dimethicone (Silikonöl 350 cs), Glyceride des Kokosfettalkohols (Novata AB), Glycerylstearat (Cutina GMS), Triglyceride der Caprinsäure und/oder Caprylsäure (Myritol 318) sowie beliebige Mischungen davon.

Die erfindungsgemäße kosmetische oder pharmazeutische Zusammensetzung kann auch weitere Bestandteile als die zuvor genannten enthalten. In einer bevorzugten Ausführungsform enthält sie mindestens eine der im folgenden aufgezählten Substanzen. Sie kann auch jede beliebige Kombination der im folgenden aufgezählten Bestandteile enthalten.

### Weitere modifizierte Polysaccharide

Neben den zuvor genannten hydrophob modifizierten Polysacchariden mit Grenzflächenaktivität kann die erfindungsgemäße Zusammensetzung auch weitere modifizierte Polysaccharide enthalten, insbesonder solche die anionische Gruppen, insbesondere Carboxylat- oder PhosphatGruppen, umfassen.

Als bevorzugte Beispiele hierfür seien Polysaccharide genannt, die durch tertiäre Amine der allgemeinen Formel HO-(CH₂)ₙ-N(CHR'-CHR-COOM)₂ und HO-(CH₂)ₙ-NR"(CHR'-CHR-COOM) modifiziert sind, mit R = H oder CH₃, R' = H, CH₃ oder COOM, R" = H oder C₁₋₁₈-Alkyl, und M ausgewählt aus der Gruppe bestehend aus Wasserstoff-, Alkali-, Erdalkali- und Ammonium-Kationen, wobei die Modifizierung des Polysaccharids vorzugsweise in Form der Ausbildung einer Ether-Bindung zwischen Hydroxygruppen des Polysaccharids und Hydroxygruppe des tertiären Amins vorliegt. In einer bevorzugten Ausführungsform handelt es sich bei dem modifizierten Polysaccharid hierbei um Stärke, also Amylose oder Amylopektin.

Als weiteres bevorzugtes Beispiel seien Polysaccharide genannt, die Hydroxyalkyl- und PhosphatGruppen tragen, wobei durch Hydroxypropyl und Phsophat modifizierte Stärke bevorzugt ist (wie beispielsweise Structure XL von National Starch).

Als weiteres bevorzugtes Beispiel seien Salze von mit alkylierten Säuren modifizierter Stärke genannt wie beispielsweise Aluminium Starch Octenylsuccinate (Dry Flo PLUS, National Starch).

### Pflanzenextrakte

In einer erfindungsgemäßen Ausführungsform enthält die Zusammensetzung mindestens einen Pflanzenextrakt. Der Pflanzenextrakt kann beispielsweise durch Extraktion der gesamten Pflanze, aber auch ausschließlich durch Extraktion aus Blüten und/oder Blättern und/oder Samen und/oder anderen Pflanzenteilen, hergestellt werden. Erfindungsgemäß sind vor allem die Extrakte aus dem Meristem, also dem teilungsfähigen Bildungsgewebe der Pflanzen, und die Extrakte aus speziellen Pflanzen wie Grünem Tee, Hamamelis, Kamille, Stiefmütterchen, Paeonie, Aloe Vera, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Fichtennadeln, Sandelholz, Wacholder, Kokosnuß, Kiwi, Guave, Limette, Mango, Aprikose, Weizen, Melone, Orange, Grapefruit, Avocado, Rosmarin, Birke, Buchensprossen, Wiesenschaumkraut, Schafgarbe, Quendel, Thymian, Melisse, Hauhechel, Eibisch (Althaea), Veilchen, Blättern der Schwarzen Johannisbeere, Huflattich, Fünffingerkraut, Ginseng, Ingwerwurzel und Süßkartoffel als weiteres Pflanzenextrakt bevorzugt. Vorteilhaft eingesetzt werden können auch Algenextrakte. Die erfindungsgemäß verwendeten Algenextrakte stammen aus Grünalgen, Braunalgen, Rotalgen oder Blaualgen (Cyanobakterien). Die zur Extraktion eingesetzten Algen können sowohl natürlichen Ursprungs als auch durch biotechnologische Prozesse gewonnen und gewünschtenfalls gegenüber der natürlichen Form verändert sein. Die Veränderung der Organismen kann gentechnisch, durch Züchtung oder durch die Kultivation in mit ausgewählten Nährstoffen angereicherten Medien erfolgen. Bevorzugte Algenextrakte stammen aus Seetang, Blaualgen, aus der Grünalge Codium tomentosum sowie aus der Braunalge Fucus vesiculosus. Ein besonders bevorzugter Algenextrakt stammt aus Blaualgen der Species Spirulina, die in einem Magnesium-angereicherten Medium kultiviert wurden.

Als Pflanzenextrakt besonders bevorzugt sind die Extrakte aus Spirulina, Grünem Tee, Ginseng, schwarzer Johannisbeere, Aloe Vera, Meristem, Hamamelis, Aprikose, Guave, Süßkartoffel, Limette, Mango, Kiwi, Gurke, Malve, Eibisch und Veilchen. Die erfindungsgemäßen Mittel können auch Mischungen aus mehreren, insbesondere aus zwei, verschiedenen Pflanzenextrakten enthalten. In einer besonders bevorzugten Ausführungsform ist in der erfindungsgemäßen Zusammensetzung einer der zuvor genannten Extrakte, insbesondere ein Extrakt aus Aloe vera, in einer Menge von 0,005 bis 0,1 Gew.-% enthalten.

In einer weiteren bevorzugten Ausführungsform enthalten die erfindungsgemäßen Zusammensetzungen einen Extrakt aus den Bohnen von Kakao (Theobroma cacao) und/oder einen Extrakt aus den Blättern der Pfefferminze (Mentha piperita).

Als Extraktionsmittel zur Herstellung der Pflanzenextrakte können beispielsweise Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol, Propylenglykol und Butylenglykol und zwar sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen. Die Wasserdampfdestillation fällt erfindungsgemäß unter die bevorzugten Extraktionsverfahren. Die Extraktion kann aber gegebenenfalls auch in Form von Trockenextraktion erfolgen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch. Je nach Wahl der Extraktionsmittel kann es bevorzugt sein, den Pflanzenextrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C₈₋₂₂-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglykolcapryl-/- /caprinsäureglyceride, Polyoxyethylenglycerinmonolaurat und Polyoxyethylenglykolkokosfettsäureglyceride.

Weiterhin kann es bevorzugt sein, in den erfindungsgemäßen Mitteln Mischungen aus mehreren, insbesondere aus zwei, verschiedenen Pflanzenextrakten einzusetzen.

Hinsichtlich der erfindungsgemäß verwendbaren Pflanzenextrakte wird weiterhin auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

### Alkohole

Die erfindungsgemäßen Zusammensetzungen können weiterhin wenigstens einen wasserlöslichen Alkohol enthalten. Unter Wasserlöslichkeit versteht man erfindungsgemäß, dass sich wenigstens 5 Gew.-% des Alkohols bei 20 °C klar lösen oder aber - im Falle langkettiger oder polymerer Alkohole - durch Erwärmen der Lösung auf 50 °C bis 60 °C in Lösung gebracht werden können. Geeignet sind je nach Darreichungsform einwertige Alkohole wie z. B. Ethanol, Propanol oder Isopropanol. Weiterhin geeignet sind wasserlösliche Polyole. Hierzu zählen wasserlösliche Diole, Triole und höherwertige Alkohole sowie Polyethylenglycole. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Hexandiole wie z. B. 1,6-Hexandiol. Weiterhin bevorzugt geeignet sind Glycerin und insbesondere Diglycerin und Triglycerin, 1,2,6-Hexantriol sowie die Dipropylenglycol und die Polyethylenglycole (PEG) PEG-400, PEG-600, PEG-1000, PEG-1550, PEG-3000 und PEG-4000.

Die Menge des Alkohols oder des Alkohol-Gemisches in den erfindungsgemäßen Zusammensetzungen beträgt, falls vorhanden, vorzugsweise 0 bis 20 Gew.-%, bezogen auf das Gesamtgewicht Zusammensetzung. Erfindungsgemäß kann sowohl ein Alkohol als auch ein Gemisch mehrerer Alkohole eingesetzt werden.

### Hydrophil modifizierte Silikone

Die erfindungsgemäße Zusammensetzung kann ferner gegebenenfalls auch hydrophil modifizierte Silikone enthalten. Unter hydrophil modifizierten Silikonen bzw. Silikon-basierten nichtionischen Emulgatoren werden erfindungsgemäß Polyorganosiloxane mit hydrophilen Substituenten verstanden, welche die Wasserlöslichkeit bzw. Grenzflächenaktivität der Silikone erhöhen. Die hydrophil modifizierten Silikone reduzieren die Klebrigkeit und hinterlassen ein frisches Hautgefühl. Unter den hydrophil modifizierten Silikonen sind solche bevorzugt, die sich mindestens in einer Menge von 0,5 Gew.-% bei 20 °C in Wasser lösen. Entsprechende hydrophile Substituenten sind beispielsweise Hydroxy-, Hydroxyalkyl-, Polyalkylenglycol-Seitenketten, insbesondere Polyethylenglycol- oder Polyethylenglycol/Polypropylenglycol-Seitenketten, sowie ethoxylierte Ester-Seitenketten.

Bevorzugte Polyorganosiloxane umfassen hierbei entsprechend zum einen dialkylierte Si-Atome, wobei die beiden Substituenten vorzugsweise unabhängig voneinander ausgewählt sind aus C₁₋₁₂-Alkyl, besonders bevorzugt C₁₋₆-Alkyl, und es sich bei den beiden Substituenten ganz besonders bevorzugt um 2 Methylreste handelt; zum anderen handelt es sich um Si-Atome, die zum einen einen Alkylsubstituenten, insbesondere C₁₋₁₂-Alkyl, vorzugsweise C₁₋₆-Alkyl, besonders bevorzugt Methyl, und zum anderen einen hydrophilen Substituenten tragen. Der hydrophile Substituent kann hierbei ausgewählt sein aus Hydroxyalkyl-Gruppen, aus Polyethergruppen, insbesondere Polyethylenglykol- und Polypropylenglykol-Gruppen sowie Polyethylenglykol/Polypropylenglykol-Misch- oder Blockcopolymer-Gruppen, sowie Alkyl-Polyether-Gruppen. Erfindungsgemäß kann das Polysiloxan auch verschiedene hydrophile Substituenten, insbesondere der zuvor genannten Arten, tragen. Der hydrophile Substituent kann hierbei über eine Alkyl-Gruppe, insbesondere eine C₁₋₆-Alkyl-Gruppe, vor allem über eine Ethylen- oder Propylen-Gruppe, an das Si-Atom gebunden sein.

Bei der Polyether-Gruppe handelt es sich vorzugsweise um eine Polyoxy-(C₁₋₄)-alkylen-Gruppe, wobei das Polyoxy-(C₁₋₄)-alkylen vorzugsweise ausgewählt ist aus Polyoxyethylen, Polyoxypropylen und Copolymere davon, wobei es sich bei dem Copolymer um ein Misch-Copolymer oder ein Block-Copolymer handeln kann, Block-Copolymere jedoch bevorzugt sind. Die Anzahl der Oxy-alkylen-Einheiten beträgt vorzugsweise von 4 bis 100. Besonders bevorzugt sind Ausführungsformen mit 4 bis 50 Oxyethylen-Einheiten und/oder mit 4 bis 50 Oxypropylen-Einheiten, insbesondere mit 4 bis 50 Oxyethylen-Einheiten, an die sich 4 bis 20 Oxypropylen-Einheiten anschließen.

Die vom Polysiloxan-Rückgrat wegweisende terminale Hydroxy-Gruppe der Polyoxyalkylene ist in einer bevorzugten Ausführungsform frei. Sie kann jedoch in anderen bevorzugten Ausführungsformen eine Ether-Bindung zu einem Alkanol, insbesondere zu einem C₁₋₂₄-Alkanol, vorzugsweise einem C₁₋₆-Alkanol oder einem C₁₆₋₂₀-Alkanol, besonders bevorzugt zu Methanol oder Ethanol, ausbilden. In einer anderen bevorzugten Ausführungsform liegt die Ausbildung einer Ester-Bindung zu einer Carbonsäure vor, wobei die Carbonsäure auch ein- oder mehrfach ungesättigt sein kann, insbesondere zu einer C₁₋₂₄-Carbonsäure, vorzugsweise zu einer C₁₂₋₂₀-Carbonsäure, besonders bevorzugt zu Essigsäure, Propansäure, Butansäure, Ölsäure, Linolsäure, Linolensäure, Palmitinsäure oder Palmitoleinsäure.

Die terminalen Si-Atome der Polysiloxane können gegebenenfalls auch zuvor genannte hydrophile Substituenten, gebunden in der zuvor genannten Art, tragen. In bevorzugten Ausführungsformen tragen nur die terminalen Si-Atome des Polysiloxan-Rückgrates hydrophile Substituenten, so dass eine blockartige Anordnung von hydrophilen Substituenten und Polysiloxan-Rückgrat vorliegt. In einer anderen bevorzugten Ausführungsform befinden sich die hydrophilen Substituenten nicht an den terminalen Si-Atomen des Polysiloxan-Rückgrates, sondern nur an den Si-Atomen im Inneren des Polysiloxan-Rückgrates, so dass eine propfartige Anordnung von hydrophilen Substituenten und Polysiloxan-Rückgrat vorliegt.

Insbesondere handelt es sich erfindungsgemäß um polymere Moleküle, umfassend Einheiten der Formel -Si(R¹)(R²)(O)- und Einheiten der Formel
-Si(R³)([X]ₘ-[Y]ₙ-[B]ₒ-[Z]ₚ-H)(O)- , wobei
R¹, R² und R³ unabhängig voneinander für C₁₋₁₂-Alkyl, insbesondere C₁₋₆-Alkyl, besonders bevorzugt Methyl, stehen,
X für C₁₋₆-Alkylen, insbesondere Ethylen oder Propylen, steht,
Y für (C₁-C₄)-Oxyalkylen steht, insbesondere für Oxyethylen und/oder Oxypropylen, wobei eine gemischte oder blockartige Anordnung vorliegen kann,
B für CO steht,
Z für C₁₋₂₄-Alkylen, vorzugsweise C₁₋₆-Alkylen oder C₁₂₋₂₀-Alkylen, besonders bevorzugt C₁₆₋₁₈-Alkylen, steht,
m 0 oder 1, bevorzugt 1, ist,
n einen Wert von 4 bis 100, besonders bevorzugt von 4 bis 50, besitzt,
o 0 oder 1 ist,
p 0 oder 1 ist.

Alle Kohlenwasserstoffreste der erfindungsgemäßen Polysiloxane können gegebenenfalls auch ein- oder mehrfach substituiert sein, insbesondere durch Reste ausgewählt aus Halogen, insbesondere Fluor, Chlor oder Brom, Hydroxy, Alkoxy, insbesondere C₁₋₆-Alkoxy, Amino, Alkylamino, insbesondere C₁₋₆-Alkylamino, Dialkylamino, insbesondere Di-C₁₋₆-Alkylamino, Aryl, insbesondere C₆₋₁₀-Aryl, Arylalkyl, insbesondere C₆₋₁₀-Aryl-C₁₋₆-alkyl und cycloaliphatischen Resten.

Es können hierbei auch unterschiedliche Ausführungsformen der zuvor genannten Einheiten im Molekül enthalten sein, die unterschiedliche Reste und/oder Substituenten aufweisen.

In einer bevorzugten Ausführungsform sind m = 1, n = 5 bis 60, o und p = 0.

Im Falle des Vorhandenseins von Alkylenoxy-Einheiten liegt das Verhältnis zwischen Alkylenoxy-Einheiten und Si-Atomen vorzugsweise zwischen 1 : 1 und 10 : 1. Die Molmasse der hydrophil modifizierten Silicone liegt vorzugsweise zwischen 200 und 10000 g/mol, besonders bevorzugt zwischen 300 und 5000 g/mol. Die Anzahl der Si-Atome mit hydrophilen Gruppen zur Gesamtzahl der Si-Atome, erfindungsgemäß auch Modifizierungsgrad genannt, liegt vorzugsweise zwischen 5 und 30 %, besonders bevorzugt zwischen 10 und 20 %.

Vorzugsweise besitzt eine 0,2%ige wäßrige Lösung der hydrophil modifzierten Silikone bei 25°C eine Oberflächenspannung von kleiner gleich 40 mN/m, besonders bevorzugt von kleiner gleich 35 mN/m.

Erfindungsgemäß geeignet sind beispielsweise hydrophil modifizierte Silicon-Copolyole, insbesondere Dimethicone-Copolyole, die beispielsweise von Wacker-Chemie unter der Bezeichnung Belsil^{®} DMC 6031 (PEG/PPG-25/25 Dimethicone), Belsil^{®} DMC 6032 (Bis-PEG-15 Methyl Ether Dimethicone), Belsil^{®} DMC 6038 (Bis-PEG-15 Methyl Ether Dimethicone) oder Belsil^{®} DMC 3071 VP, von Dow Corning unter der Bezeichnung DC 193, DC 5324 oder DC 5329 (jeweils PEG-12 Dimethicone), DC 2501 (BIS-PEG-18 Methyl Ether Dimethyl Silane) oder DC Q2-5220 (PEG/PPG-17/18 Dimethicone), von Degussa/Goldschmidt unter der Bezeichnung Abil EM 90 (Cetyl PEG/PPG-10/1 Dimethicone), Abil EM 97 (Bis-PEG/PPG-14/14 Dimethicone), Abil Care 85 (Bis-PEG/PPG-16/16 Dimethicone; Caprylic/Capric Triglyceride), Abil B 8832 (Bis-PEG/PPG-20/20-Dimethicone), Abil B 88183 oder Abil B 88184 (jeweils PEG/PPG-20/6-Dimethicone), von Noveon unter der Bezeichnung Ultrasil DW-18 (Dimethicone PEG-7 Isostearate), Ultrasil SW-12 (Dimethicone PEG-7 Cocoate), Ultrasil DW-AV (Dimethicone PEG-7 Avocadoate) oder Ultrasil DW-O (Dimethicone PEG-7 Olivate) und von Shin-Etsu unter der Bezeichnung KF618 (PEG-6 Methyl Ether Dimethicone), KF6011 (PEG-11 Methyl Ether Dimethicone), KF6012 (PEG/PPG-20/22 Butyl Ether Dimethicone), KF6013 (PEG-9 Dimethicone), KF6015 (PEG-3 Dimethicone), KF6016 (PEG-9 Methyl Ether Dimethicone) oder KF6017 (PEG-10 Dimethicone) im Handel erhältlich sind. Erfindungsgemäß kann auch ein beliebiges Gemisch der genannten Silicone eingesetzt werden.

Die Menge des hydrophil modifizierten Silicons in den erfindungsgemäßen Zusammensetzungen beträgt vorzugsweise 0,1 - 5,0 Gew.-%, besonders bevorzugt 0,5 - 4,0 Gew.-%, insbesondere 1,0 - 3,0 Gew.-%, bezogen auf das Gesamtgewicht der Zusammensetzung.

### Aminosäuren, Peptide, Proteine und deren Derivate

Weiterhin können die erfindungsgemäßen Zusammensetzungen Monomere, Oligomere und Polymere von Aminosäuren und N-C₂-C₂₄-Acylaminosäuren sowie Ester und/oder physiologisch verträgliche Metallsalze dieser Substanzen enthalten.

Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, , Threonin, Thyroxin, Tryptophan, Tyrosin, Valin, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, , Zink- und Natriumpyroglutamat und Natriumlauroylglutamat.

Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Die Oligomere der Aminosäuren und/oder der N-C₂-C₂₄-Acylaminosäuren sind ausgewählt aus Di-, Tri-, Tetra-, Penta-, Hexa- oder Pentadecapeptiden, die acyliert und/oder verestert sein können. Erfindungsgemäß bevorzugte, gegebenenfalls acylierte und/oder veresterte Dipeptide sind Tyr-Arg (Dipeptide-1), Val-Trp (Dipeptide-2), Asn-Phe, Asp-Phe, N-Palmitoyl-β-Ala-His, N-Acetyl-Tyr-Arghexyldecylester (z. B. Calmosensine von Sederma), Carnosin (ß-Ala-His) und N-Palmitoyl-Pro-Arg. Erfindungsgemäß bevorzugte, gegebenenfalls acylierte und/oder veresterte Tripeptide sind Lys-Pro-Val, Gly-His-Lys (Tripeptide-1, z. B. Omega-CH-Aktivator von GfN), Palmitoyl-Arg-Phe-Lys (Syn-Coll, Pentapharm), N-Palmitoyl-Gly-His-Lys, Gly-Lys-His, Tyr-Tyr-Val, Tyr-Val-Tyr, Val-Tyr-Val (Tripeptide-2), Gly-His-Arg (Tripeptide-3), N-Myristoyl-Gly-His-Arg (z. B. Collasyn 314-GR von Therapeutic Peptide Inc.), Tripeptide-4 (z. B. ATPeptide, zu beziehen über IMPAG), His-Ala-Orn, Lys-Phe-Lys, N-Elaidoyl-Lys-Phe-Lys und N-Acetyl-Arg-Lys-Arg-NH₂. Erfindungsgemäß bevorzugte, gegebenenfalls acylierte und/oder veresterte Tetrapeptide sind Val-Val-Arg-Pro, Gly-Gln-Pro-Arg, Gly-Gln-Arg-Pro und N-Palmitoyl-Gly-Gln-Pro-Arg. Erfindungsgemäß bevorzugtes gegebenenfalls acylierte und/oder veresterte Pentapeptide sind Lys-Thr-Thr-Lys-Ser, N-Palmitoyl-Lys-Thr-Thr-Lys-Ser, N-Palmitoyl-Tyr-Gly-Gly-Phe-Met, Val-Val-Arg-Pro-Pro und N-Palmitoyl-Tyr-Gly-Gly-Phe-Leu. Erfindungsgemäß bevorzugte, gegebenenfalls acylierte und/oder veresterte Hexapeptide sind Palmitoyl-Val-Gly-Val-Ala-Pro-Gly (Biopeptide EL von Sederma), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (Z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1).

Es kann erfindungsgemäß besonders bevorzugt sein, ein Gemisch aus mindestens zwei Oligopeptiden einzusetzen. Ein besonders bevorzugtes Gemisch ist die Kombination aus N-Palmitoyl-Gly-His-Lys (z.B. Biopeptide CL von Sederma) und N-Palmitoyl-Gly-Gln-Pro-Arg (z. B. in Eyeliss von Sederma). Eine vorgefertigte Mischung des Tripeptids Palmitoyl-Gly-His-Lys und des Tetrapeptids N-Palmitoyl-Gly-Gln-Pro-Arg ist unter dem Handelsnamen Matrixyl 3000, ebenfalls von Sederma, erhältlich und ist erfindungsgemäß ebenfalls besonders bevorzugt.

Die Polymere der Aminosäuren und/oder der N-C₂-C₂₄-Acylaminosäuren sind ausgewählt aus pflanzlichen und tierischen Proteinhydrolysaten und/oder Proteinen. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, z. B. die Handelsprodukte Phytokine von Coletica oder Ridulisse C von Silab. Proteinhydrolysate können naturgemäß auch monomere Aminosäuren und Oligopeptide enthalten; ihre Zusammensetzung ist normalerweise nicht definiert.
Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).
Erfindungsgemäß einsetzbar sind auch kationisierte Proteinhydrolysate. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.

In einer weiteren bevorzugten Ausführungsform sind die Polymeren der Aminosäuren ausgewählt aus DNA-Reparaturenzymen.
Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen. Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.
Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.
In den erfindungsgemäßen Zusammensetzungen sind die Photosome™ oder Ultrasome™ in Mengen von 0,1 - 10 Gew.-%, bevorzugt 0,5 - 5,0 Gew.-% und besonders bevorzugt 1,0 - 4,0 Gew.-%, bezogen auf das gesamte Mittel, enthalten. In den erfindungsgemäßen Zusammensetzungen sind die Monomere, Oligomere oder Polymere von Aminosäuren, N-C₂-C₂₄-Acylaminosäuren und/oder den Estern und/oder den physiologisch verträglichen Metallsalzen dieser Substanzen in Mengen von 0,01 - 10 Gew.-%, bevorzugt 0,1 - 5 Gew.-% und besonders bevorzugt 0,1 - 3 Gew.-%, jeweils bezogen auf die gesamte Zusammensetzung, enthalten.

In einer weiteren bevorzugten Ausführungsform liegen die Monomeren, Oligomeren und Polymeren von Aminosäuren, N-C₂-C₂₄-Acylaminosäuren, den Estern und/oder den physiologisch verträglichen Metallsalzen dieser Substanzen in geträgerter Form vor, insbesondere aufgetragen auf feinteiligen, pulverförmigen Substraten wie Kieselgel, insbesondere Aerosil-Typen, Talkum, Microsponges, modifizierten Stärken und Stärkederivaten, kristalliner Cellulose, Cellulosepulvern, Lactoglobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

### Oligonucleotide

Die erfindungsgemäßen Zusammensetzungen können weiterhin Oligonukleotide enthalten.

Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).

Ein erfindungsgemäß besonders bevorzugtes Oligonucleotid ist das Thymidin-Dinucleotid.

In den erfindungsgemäßen Zusammensetzungen sind die DNA-Oligonucleotide oder RNA-Oligonucleotide in Mengen von 0,0001 - 5 Gew.-%, bevorzugt 0,001 - 1,0 Gew.-% und besonders bevorzugt 0,01 - 0,5 Gew.-%, bezogen auf das gesamte Mittel, enthalten.

### Betain-Verbindungen

Die erfindungsgemäßen Zusammensetzungen können weiterhin Betain-Verbindungen enthalten.

Erfindungsgemäß eingesetzte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl.

Die Betainverbindungen sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

### Zuckerbestandteile

Weiterhin können die erfindungsgemäßen Zusammensetzungen mindestens ein Mono-, Oligo- oder Polysaccharid oder deren Derivate enthalten.

Erfindungsgemäß geeignete Monosaccharide sind z. B. Glucose, Fructose, Galactose, Arabinose, Ribose, Xylose, Lyxose, Allose, Altrose, Mannose, Gulose, Idose und Talose, die Desoxyzucker Fucose und Rhamnose sowie Aminozucker wie z. B. Glucosamin oder Galactosamin. Bevorzugt sind Glucose, Fructose, Galactose, Arabinose und Fucose; Glucose ist besonders bevorzugt.

Erfindungsgemäß geeignete Oligosaccharide sind aus zwei bis zehn Monosaccharideinheiten zusammengesetzt, z. B. Saccharose, Lactose oder Trehalose. Ein besonders bevorzugtes Oligosaccharid ist Saccharose. Ebenfalls besonders bevorzugt ist die Verwendung von Honig, der überwiegend Glucose und Saccharose enthält.

Erfindungsgemäß geeignete Polysaccharide sind aus mehr als zehn Monosaccharideinheiten zusammengesetzt. Bevorzugte Polysaccharide sind die aus α-D-Glucose-Einheiten aufgebauten Stärken sowie Stärkeabbauprodukte wie Amylose, Amylopektin und Dextrine. Erfindungsgemäß besonders vorteilhaft sind chemisch und/oder thermisch modifizierte Stärken, z. B. Hydroxypropylstärkephosphat, Dihydroxypropyldistärkephosphat oder die Handelsprodukte Dry Flo^{®}. Weiterhin bevorzugt sind Dextrane sowie ihre Derivate, z. B. Dextransulfat. Ebenfalls bevorzugt sind nichtionische Cellulose-Derivate, wie Methylcellulose, Hydroxypropylcellulose oder Hydroxyethylcellulose, sowie kationische Cellulose-Derivate, z. B. die Handelsprodukte Celquat^{®} und Polymer JR^{®}, und bevorzugt Celquat^{®} H 100, Celquat^{®} L 200 und Polymer JR^{®} 400 (Polyquaternium-10) sowie Polyquaternium-24. Weitere bevorzugte Beispiele sind Polysaccharide aus Fucose-Einheiten, z. B. das Handelsprodukt Fucogel^{®}. Besonders bevorzugt sind die aus Aminozuckereinheiten aufgebauten Polysaccharide, insbesondere Chitine und ihre deacetylierten Derivate, die Chitosane, und Mucopolysaccharide. Zu den erfindungsgemäß bevorzugten Mucopolysacchariden gehören Hyaluronsäure und ihre Derivate, z. B. Natriumhyaluronat oder Dimethylsilanolhyaluronat, sowie Chondroitin und seine Derivate, z. B. Chondroitinsulfat.

Die Zuckerbestandteile sind, falls vorhanden, vorzugsweise in Mengen bis zu 5 Gew.-% in den erfindungsgemäßen Zusammensetzungen enthalten.

### Weitere Polymere

Außer den hydrophob modifizierten Polymeren können weitere Polymere, insbesondere ausgewählt aus kationischen, anionischen und nichtionischen Polymeren enthalten sein.

Unter den.kationischen Polymeren bevorzugt sind Polysiloxane mit quaternären Gruppen, z. B. die Handelsprodukte Q2-7224 (Dow Corning), Dow Corning^{®} 929 Emulsion (mit Amodimethicone), SM-2059 (General Electric), SLM-55067 (Wacker) sowie Abil^{®}-Quat 3270 und 3272 (Th. Goldschmidt).

Bevorzugte anionische Polymere, die die Wirkung des erfindungsgemäß verwendeten Wirkstoffs unterstützen können, enthalten Carboxylat- und/oder Sulfonatgruppen und als Monomere zum Beispiel Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure. Ganz besonders bevorzugte anionische Polymere enthalten als alleiniges Monomer oder als Comonomer 2-Acrylamido-2-methylpropansulfonsäure, wobei die Sulfonsäuregruppe ganz oder teilweise in Salzform vorliegen kann. Innerhalb dieser Ausführungsform ist es bevorzugt, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppenhaltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppen ganz oder teilweise als Natrium-, Kalium-, Ammonium-, Mono- oder Triethanolammonium-Salz vorliegen. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Weitere bevorzugte anionische Homo- und Copolymere sind unvernetzte und vernetzte Polyacrylsäuren. Dabei können Allylether von Pentaerythrit, von Sucrose und von Propylen bevorzugte Vernetzungsagentien sein.

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Die genannten weiteren Polymere sind, falls vorhanden, vorzugsweise in Mengen bis zu 2 Gew.-% enthalten.

### Hydroxy- und Ketocarbonsäuren

In einer weiteren bevorzugten Ausführungsform enthalten die erfindungsgemäßen Zusammensetzungen mindestens eine α-Hydroxycarbonsäure, α-Ketocarbonsäure oder β-Hydroxycarbonsäure oder deren Ester-, Lacton- oder Salzform. Erfindungsgemäß geeignete α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren, α-Ketocarbonsäuren oder β-Hydroxycarbonsäuren oder ihre Derivate sind in Mengen von 0,1 - 10 Gew.-%, bevorzugt 0,5 - 5 Gew.-%, jeweils bezogen auf die gesamte Zusammensetzung, enthalten.

### Verdickungsmittel

Flüssige und gelförmige Darreichungsformen der erfindungsgemäßen Zusammensetzungen können weitere Verdickungsmittel enthalten, z. B. Celluloseether, wie Hydroxypropylcellulose, Hydroxyethylcellulose und Methylhydroxypropylcellulose, des weiteren Gelatine, Pflanzengumme wie Agar-Agar, Guar-Gum, Alginate, Xanthan-Gum, Gummi arabicum, Karaya-Gummi oder Johannisbrotkernmehl, sowie verdickende Polymere auf Basis von Polyacrylaten, die gewünschtenfalls vernetzt sein können, oder auf Basis von Polyacrylamiden oder sulfonsäuregruppenhaltigen Polyacrylaten, z. B Sepigel^{®} 305 oder Simulgel^{®} EG, weiterhin anorganische Verdicker, z. B. natürliche und synthetische Tone und Schichtsilikate, z. B. Bentonit, Hectorit, Montmorillonit oder Laponite^{®}, sowie vollsynthetische Hydrokolloide wie z. B. Polyvinylalkohol, und außerdem Ca-, Mg- oder Zn-Seifen von Fettsäuren.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin; Dialkoholamine, wie Diethanolamin oder 2-Amino-1,3-propandiol. In einer erfindungsgemäß bevorzugten Ausführungsform wird Glycerin in einer Menge von 2 bis 5 Gew.-% eingesetzt.

### Konservierungsmittel

Als Konservierungsmittel eignen sich beispielsweise Phenoxyethanol, Formaldehydlösung, Parabene, insbesondere Methyl- oder Propylparaben, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-toluamid, 1,2-Pentandiol oder Ethyl Butylacetylaminopropionate in Frage, als Selbstbräuner eignet sich Dihydroxyaceton.

### Vitamine

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen Zubereitungen enthalten die Vitamin A-Komponente bevorzugt in Mengen von 0,05 - 1 Gew.-%, bezogen auf die gesamte Zusammensetzung.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf das gesamte Mittel, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf das gesamte Mittel, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Vitamin C (Ascorbinsäure) wird bevorzugt in Mengen von 0,1 bis 3 Gew.-%, bezogen auf die gesamte Zusammensetzung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.%, bezogen auf die gesamte Zusammensetzung, enthalten.

Unter Vitamin F werden üblicherweise essentielle Fettsäuren, insbesondere Linolsäure, Linolensäure und Arachidonsäure, verstanden.

Vitamin H ist eine andere Bezeichnung für Biotin oder Vitamin B₇ (siehe oben).

Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.

Vitamin A-palmitat (Retinylpalmitat), Panthenol, , Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

### Flavonoide

In einer weiteren bevorzugten Ausführungsform enthalten die erfindungsgemäßen Zusammensetzungen mindestens ein Flavonoid oder mindestens einen Flavonoid-reichen Pflanzenextrakt.

Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Gtucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglu-cosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).

Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.

Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

### Isoflavonoide

In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt.

Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin. Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoidreicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.

Erfindungsgemäß werden die Isoflavonoide in Mengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, eingesetzt.

### Polyphenole

In einer weiteren bevorzugten Ausführungsform enthalten die erfindungsgemäßen Zusammensetzungen mindestens ein Polyphenol oder einen Polyphenol-reichen Pflanzenextrakt.

Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay, ein Extrakt aus den Kernen der Chardonnay-Traube, erhältlich von der Firma Croda. Erfindungsgemäß werden die Polyphenole in Mengen von 0,001 bis 10 Gew.-%, bevorzugt 0,005 bis 5 Gew.-% und besonders bevorzugt 0,01 bis 3 Gew.-%, jeweils bezogen auf die gesamte kosmetische Zusammensetzung, eingesetzt.

### Ubichinol-Verbindungen

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (II) auf: 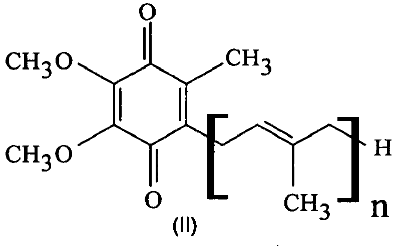 mit n = 6, 7, 8, 9 oder 10.

Besonders bevorzugt ist das Ubichinon der Formel (II) mit n = 10, auch bekannt als Coenzym Q10. Erfindungsgemäß werden die Ubichinone, Ubichinole oder deren Derivate in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

### UV-Filtersubstanzen

In einer weiteren bevorzugten Ausführungsform enthalten die erfindungsgemäßen Zusammensetzungen mindestens eine anorganische und/oder mindestens eine organische UV-Filtersubstanz.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.

Die erfindungsgemäß verwendeten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/ Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS-Nr.: 025973-551), 2-(2'- Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2- propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis- {[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

### Sebumregulierende Wirkstoffe

In einer weiteren bevorzugten Ausführungsform enthalten die erfindungsgemäßen Zusammensetzungen mindestens einen sebumregulierenden Wirkstoff. Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques) und den Wirkstoffmischungen Asebiol^{®} BT 2 (Laboratoires Sérobiologiques, INCI: Aqua, Hydrolyzed Yeast Protein, Pyridoxine, Niacinamide, Glycerin, Panthenol, , Biotin) und Antifettfaktor^{®} COS-218/2-A (Cosmetochem, INCI: Aqua, Cetyl-PCA, PEG-8 Isolauryl Thioether, PCA, Cetyl Alcohol).

Die sebumregulierenden Wirkstoffe sind in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

### Deodorantien

Die erfindungsgemäßen Zusammensetzungen enthalten in einer besonderen erfindungsgemäßen Ausführungsform mindestens einen Deodorant-Wirkstoff. Erfindungsgemäß als Deodorant-Wirkstoffe geeignet sind Duftstoffe, antimikrobielle, antibakterielle oder keimhemmende Stoffe, enzymhemmende Stoffe, Antioxidantien und Geruchsadsorbentien.

Geeignet sind insbesondere Organohalogenverbindungen sowie -halogenide, quartäre Ammoniumverbindungen und Zinkverbindungen. Bevorzugt sind Chlorhexidin und Chlorhexidingluconat, Benzalkoniumhalogenide und Cetylpyridiniumchlorid. Desweiteren sind Natriumbicarbonat, Natriumphenolsulfonat und Zinkphenolsulfonat, die Bestandteile des Lindenblütenöls, Phenoxyethanol, Triclosan (Irgasan® DP300) oder Triethylcitrat einsetzbar.

Als Enzym-hemmende Stoffe bevorzugt sind Inhibitoren für Enzyme der axillaren Keimflora, die bei der Entstehung von Körpergeruch involviert sind. Hierbei handelt es sich vorzugsweise um Inhibitoren von Lipasen, Arylsulfatasen (s. WO 01/99376), β-Glucoronidasen (s. WO 03/039505), 5-α-Reduktasen und Aminoacylasen.

Weitere antibakteriell wirksame Deodorant-Wirkstoffe sind Parfümöle, Ethylhexylglycerinether (Sensiva® SC 50), Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide.

Weiterhin geeignet als Deodorant-Wirkstoff sind wasserlösliche Polyole, ausgewählt aus wasserlöslichen Diolen, Triolen und höherwertigen Alkoholen sowie Polyethylenglycolen. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Pentandiole, z. B. 1,2-Pentandiol, sowie Hexandiole, z. B. 1,6-Hexandiol. Weiterhin bevorzugt geeignet sind Glycerin und technische Oligoglyceringemische mit einem Eigenkondensationsgrad von 1,5 bis 10 wie etwa technische Diglyceringemische mit einem Diglyceringehalt von 40 bis 50 Gew.-% oder Triglycerin, weiterhin 1,2,6-Hexantriol sowie Polyethylenglycole (PEG) mit einem durchschnittlichen Molekulargewicht von 100 bis 1.000 Dalton, beispielsweise PEG-400, PEG-600 oder PEG-1000. Weitere geeignete höherwertige Alkohole sind die C₄-, C₅- und C₆-Monosaccharide und die entsprechenden Zuckeralkohole, z. B. Mannit oder Sorbit.

### Antioxidantien

Die erfindungsgemäßen Zusammensetzungen können ferner Antioxidantien enthalten, zum Beispiel Imidazole (z. B. Urocaninsäure) und deren Derivate, Peptide wie D,L-Carnosin, D-Carnosin, L-Carnosin und deren Derivate (z. B. Anserin), Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, das Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Katalase, Superoxid-Dismutase, Zink und dessen Derivate (z. B. ZnO, ZnSO₄), Selen und dessen Derivate (z. B. Selen-Methionin), Stilbene und deren Derivate (z. B. Stilbenoxid, trans-Stilbenoxid) und die als Antioxidans geeigneten Derivate (Salze, Ester, Ether, Zucker, Nukleotide, Nukleoside, Peptide und Lipide) dieser Wirkstoffe.

### Weitere Wirk-, Hilfs- und Zusatzstoffe

Die erfindungsgemäßen Mittel können weitere Wirk-, Hilfs- und Zusatzstoffe enthalten, beispielsweise Taurin; Caomint (Solabia); Allantoin; Bisabolol; Ceramide und Pseudoceramide; Triterpene, insbesondere Triterpensäuren wie Ursolsäure, Rosmarinsäure, Betulinsäure, Boswelliasäure und Bryonolsäure; monomere Catechine, besonders Catechin und Epicatechin, Leukoanthocyanidine, Catechinpolymere (Catechin-Gerbstoffe) sowie Gallotannine; Pflanzenglycoside; Strukturanten wie Maleinsäure und Milchsäure; Dimethylisosorbid; Alpha-, betasowie gamma-Cyclodextrine, insbesondere zur Stabilisierung von Retinol; Lösungsmittel, Quell- und Penetrationsstoffe wie Ethanol, Isopropanol, Ethylenglykol, Propylenglykol, Propylenglykolmonoethylether, Glycerin und Diethylenglykol, Carbonate, Hydrogencarbonate, Guanidine, Harnstoffe sowie primäre, sekundäre und tertiäre Phosphate; Parfümöle, Pigmente sowie Farbstoffe zum Anfärben des Mittels; Substanzen zur Einstellung des pH-Wertes, z. B. α- und β-Hydroxycarbonsäuren; Komplexbildner wie EDTA, NTA, β-Alanindiessigsäure und Phosphonsäuren; Trübungsmittel wie Latex, Styrol/PVP- und Styrol/Acrylamid-Copolymere; Perlglanzmittel wie Ethylenglykolmono- und -distearat sowie PEG-3-distearat; Treibmittel wie Propan-Butan-Gemische, N₂O, Dimethylether, CO₂ und Luft; MMP-1-inhibierende Substanzen, insbesondere ausgewählt aus Photolyase und/oder T4 Endonuclease V, Propylgallat, Precocenen, 6-Hydroxy-7-methoxy-2,2-dimethyl-1(2H)-benzopyran und 3,4-Dihydro-6-hydroxy-7-methoxy-2,2-dimethyl-1(2H)-benzopyran; entzündungshemmende Substanzen; Feststoffe, ausgewählt aus Kieselsäuren, z. B. Aerosil^{®}-Typen, Kieselgelen, Siliciumdioxid, Tonen, z. B. Bentonite oder Kaolin, Magnesiumaluminiumsilikaten, z. B. Talkum, Bornitrid, Titandioxid, das gewünschtenfalls beschichtet sein kann, gegebenenfalls modifizierten Stärken und Stärkederivaten, Cellulosepulvern und Polymerpulvern; Anti-Akne-Wirkstoffe sowie Keratolytika.

**Ausführungsbeispiele**

| Rohstoff (INCI) | Produktname | Gehalt in Gew.-% | | | | | | |
|---|---|---|---|---|---|---|---|---|
| Cetyl Hydroxyethylcellulose | Natrosol Plus CS 330 | 2,5 | | | | | | |
| Inulin Lauryl Carbamate | Inutec SP1 | | 2,5 | 0,5 | 0,5 | 0,5 | 0,5 | 0,4 |
| Ammonium Acryloyldimethyltaurate/ VP Copolymer | Aristoflex AVC | 1,0 | 1,0 | 1,2 | 1,2 | 0,7 | 1,4 | 1,4 |
| Dicaprylyl Ether | Cetiol OE | 4,0 | 4,0 | | | | | |
| Butyrospermum Parkii | Cetiol SB 45 | 3,0 | 3,0 | 2,0 | 2,0 | 2,0 | 2,0 | 2,0 |
| Prunus Amygdalus Dulcis | Mandelöl süß | 2,0 | 2,0 | | | | | |
| Dimethicone | Silikonöl 350 cs | 1,0 | 1,0 | | | | | |
| Decyl Oleate | Cetiol V | | | 3,0 | 3,0 | 3,0 | 3,0 | 3,0 |
| Caprylic/Capric Triglyceride | Myritol 318 | | | 3,0 | 3,0 | 3,0 | 3,0 | 3,0 |
| Cetearyl Isononanoate | Cetiol SN | | | 2,5 | 2,5 | 2,5 | 2,5 | 2,5 |
| Glyceryl Stearate | Cutina GMS | | | 2,5 | 2,5 | 2,5 | 2,5 | 2,5 |
| Pentaerythrityl Distearate | Cutina PES | | | | 2,5 | | 1,0 | 1,0 |
| Cocoglycerides | Novata AB | | | 5,0 | 2,5 | | 4,0 | 4,0 |
| Jojoba Esters | Floraester 70 | 2,0 | 2,0 | | | | | |
| Jojoba Esters | Floraester 60 | 1,5 | 1,5 | | | | | |
| Aluminium Starch Octenylsuccinate | Dry Flo Plus | | | | | | | 1,0 |
| Hydroxypropyl Starch Phospahte | Structure XL | | | | | | | 1,0 |
| Glycerin | | 3,0 | 3,0 | 4,0 | 4,0 | 4,0 | 4,0 | 4,0 |
| Dipropylene Glycol | | 6,0 | 6,0 | | | | | |
| Preservative | | q.s. | q.s. | q.s. | q.s. | q.s. | q.s. | q.s. |
| Parfüme | | q.s. | q.s. | q.s. | q.s. | q.s. | q.s. | q.s. |
| Water (VE) | | ad. 100 | ad. 100 | ad. 100 | ad. 100 | ad. 100 | ad. 100 | ad. 100 |

## Patentansprüche

1. O/W-Emulsion, enthaltend folgende Bestandteile:
a)mindestens ein hydrophob modifiziertes Polysaccharid
b)0,5 bis 5,0 Gew.-% mindestens eines Copolymers basierend auf einer Acrylamidoalkansäure,
c) mindestens einen Fettstoff,
d)Wasser,
wobei es sich bei dem Copolymer gemäß b) um ein Copolymer auf Basis einer Acrylamidoalkansäure um Polymere umfassend Einheiten der Formel -CH₂-C(R¹)(W)- und Einheiten der Formel -CH₂-C(R²)([X]ₘ-[Y]ₙ-Z)- und/oder Einheiten der Formel -CH₂-C(R⁵)(Het)- handelt, wobei
R¹, R² und R⁵ unabhängig voneinander für Wasserstoff oder Methyl stehen,
W für CONR³R⁴ steht,
R³ für (C₁-C₆)-Alkyl-CO₂H, (C₁-C₆)-Alkyl-PO₃H oder (C₁-C₆)-Alkyl-SO₃H steht,
R⁴ für Wasserstoff oder (C₁-C₆)-Alkyl steht,
X für C(O)O-, C(O)NH- oder (C₁-C₆)-Alkylen steht,
Y für (C₁-C₄)-Alkylen-oxy steht,
Z für (C₅-C₅₀)-Alkyl steht
m für 0 oder 1 steht,
Het für einen N-Heterocyclus mit 5 oder 6 Ringgliedern und 1 oder 2 Heteroatomen steht,
wobei mindestens 1 Heteroatom N ist und das andere Heteroatom ausgewählt ist aus N, O oder S, und über das mindestens eine N-Heteroatom des Heterocyclus vorzugsweise Bindung an das C-Atom des Rückgrats des Polymers erfolgt, und wobei im Heterocyclus vorzugsweise mindestens eine CO-Gruppe enthalten ist, die vorzugsweise in ortho-Stellung zu dem mindestens einen N-Heteroatom angeordnet ist, und wobei der Heterocyclus gegenbenenfalls auch weitere Substituenten, insbesondere ausgewählt aus C₁₋₆-Alkyl und Halogen, tragen kann, und wobei die Kohlenwasserstoffreste gegebenenfalls auch ein- oder mehrfach substituiert sein können, insbesondere durch Reste ausgewählt aus Halogen, Hydroxy, Alkoxy, Amino, Alkylamino, Dialkylamino, Aryl, Arylalkyl und cycloaliphatischen Resten,
**dadurch gekennzeichnet, daß**
es sich bei dem hydrophob modifizierten Polysaccharid um Inulin Lauryl Carbamat oder Cetyl Hydroxyethylcellulose oder Mischungen davon handelt und es sich um eine emulgatorfreie Zubereitung handelt.

2. O/W-Emulsion, enthaltend folgende Bestandteile:
a)mindestens ein hydrophob modifiziertes Polysaccharid
b)0,5 bis 5,0 Gew.-% mindestens eines Copolymers basierend auf einer Acrylamidoalkansäure,
c)mindestens einen Fettstoff,
d)Wasser,
wobei es sich bei dem Copolymer gemäß b) um ein Copolymer auf Basis einer Acrylamidoalkansäure um Polymere umfassend Einheiten der Formel -CH₂-C(R¹)(W)- und Einheiten der Formel -CH₂-C(R²)([X]ₘ-[Y]ₙ-Z)- und/oder Einheiten der Formel -CH₂-C(R⁵)(Het)- handelt, wobei
R¹, R² und R⁵ unabhängig voneinander für Wasserstoff oder Methyl stehen,
W für CONR³R⁴ steht,
R³ für (C₁-C₆)-Alkyl-CO₂H, (C₁-C₆)-Alkyl-PO₃H oder (C₁-C₆)-Alkyl-SO₃H steht,
R⁴ für Wasserstoff oder (C₁-C₆)-Alkyl steht,
X für C(O)O-, C(O)NH- oder (C₁-C₆)-Alkylen steht,
Y für (C₁-C₄)-Alkylen-oxy steht,
Z für (C₅-C₅₀)-Alkyl steht
m für 0 oder 1 steht,
Het für einen N-Heterocyclus mit 5 oder 6 Ringgliedern und 1 oder 2 Heteroatomen steht,
wobei mindestens 1 Heteroatom N ist und das andere Heteroatom ausgewählt ist aus N, O oder S, und über das mindestens eine N-Heteroatom des Heterocyclus vorzugsweise Bindung an das C-Atom des Rückgrats des Polymers erfolgt, und wobei im Heterocyclus vorzugsweise mindestens eine CO-Gruppe enthalten ist, die vorzugsweise in ortho-Stellung zu dem mindestens einen N-Heteroatom angeordnet ist, und wobei der Heterocyclus gegenbenenfalls auch weitere Substituenten, insbesondere ausgewählt aus C₁₋₆-Alkyl und Halogen, tragen kann, und wobei die Kohlenwasserstoffreste gegebenenfalls auch ein- oder mehrfach substituiert sein können, insbesondere durch Reste ausgewählt aus Halogen, Hydroxy, Alkoxy, Amino, Alkylamino, Dialkylamino, Aryl, Arylalkyl und cycloaliphatischen Resten,
**dadurch gekennzeichnet, daß**
es sich bei dem hydrophob modifizierten Polysaccharid um Inulin Lauryl Carbamat handelt.

3. O/W-Emulsion nach Anspruch 1 oder 2, **dadurch gekennzeichnet, dass** das hydrophob modifizierte Polysaccharid in einer Menge von 0,1-3,0 Gew.-% enthalten ist.

4. O/W-Emulsion nach einem der Ansprüche 1 bis 3, **dadurch gekennzeichnet, dass** der Fettstoff in einer Menge von 5,0-30,0 Gew.-% enthalten ist.

5. O/W-Emulsion nach einem der Ansprüche 1 bis 4, **dadurch gekennzeichnet, dass** eine 1%ige wäßrige Lösung des hydrophob modifizierten Polysaccharids eine Oberflächenspannung von unter 60 mN/m hat.

6. O/W-Emulsion nach einem der Ansprüche 1 bis 5, **dadurch gekennzeichnet, dass** eine 1%ige wäßrige Lösung des hydrophob modifizierten Polysaccharids eine Grenzflächenspannung gegen die Ölphase von unter 25 mN/m hat.

7. O/W-Emulsion nach einem der Ansprüche 1 bis 6, **dadurch gekennzeichnet, dass** es sich bei der Acrylamidoalkansäure um eine Acrylamidosulfonsäure handelt.

8. O/W-Emulsion nach Anspruch 7, **dadurch gekennzeichnet, dass** es sich bei dem Copolymer auf Basis einer Acrylamidoalkansäure um Ammonium Acryloyldimethyltaurat/VP Copolymer handelt.

9. O/W-Emulsion nach einem der Ansprüche 1 bis 8, **dadurch gekennzeichnet, dass** es sich bei dem Fettstoff um ein polares oder unpolares flüssiges Öl oder um Mischungen davon handelt.

10. O/W-Emulsion nach einem der Ansprüche 1 bis 9, **dadurch gekennzeichnet, dass** sie bei einer Temperatur von 25°C eine Viskosität von 500 bis 10000 Pas bei einer Scherrate von 0,1 s⁻¹ und eine Viskosität von 1 bis 10 Pas bei einer Scherrate von 100 s⁻¹ aufweist.

11. O/W-Emulsion nach einem der Ansprüche 2 bis 10, **dadurch gekennzeichnet, dass** es sich um eine emulgatorfreie Zubereitung handelt.

12. Verwendung einer O/W-Emulsion nach einem der Ansprüche 1 bis 11 zur Behandlung der Gesichtshaut.

13. Verwendung einer O/W-Emulsion nach einem der Ansprüche 1 bis 11 zur Behandlung von empfindlicher oder trockener Haut.

14. Verwendung einer O/W-Emulsion nach einem der Ansprüche 1 bis 11 zur Vermeidung von empfindlicher oder trockener Haut und/oder zur Vermeidung von Hautrötungen und Hautirritationen.

15. Verwendung einer O/W-Emulsion nach einem der Ansprüche 1 bis 11 zur Behandlung reifer Haut.

16. Verwendung einer O/W-Emulsion nach einem der Ansprüche 1 bis 11 zur Behandlung von Haut in empfindlichen oder sensitiven Bereichen, insbesondere im Bereich der Augen.

17. Verwendung einer O/W-Emulsion nach einem der Ansprüche 1 bis 11 zur Behandlung von gestresster, insbesondere UV-gestresster, Haut.

18. Verwendung einer O/W-Emulsion nach einem der Ansprüche 1 bis 11 zur Behandlung der Haut nach einer chemischen oder mechanischen Peeling-Behandlung.

19. Verwendung einer O/W-Emulsion nach einem der Ansprüche 1 bis 11 zur Herstellung einer Zusammensetzung zum Transport von Wirkstoffen in die Haut.

## Claims

1. An O/W emulsion containing the following components:
a) at least one hydrophobically modified polysaccharide,
b) from 0.5 to 5.0 wt.% of at least one acrylamido-alkanoic-acid-based copolymer,
c) at least one fatty substance,
d) water,
the copolymer according to b) being an acrylamido-alkanoic-acid-based copolymer, being polymers comprising units of formula -CH₂-C(R¹)(W)- and units of formula -CH₂-C(R²)([X]ₘ-[Y]ₙ-Z)- and/or units of formula -CH₂-C(R⁵)(Het)-, where
R¹, R² and R⁵ represent, independently of one another, hydrogen or methyl,
W represents CONR³R⁴,
R³ represents (C₁-C₆)-alkyl-CO₂H, (C₁-C₆)-alkyl-PO₃H or (C₁-C₆)-alkyl-SO₃H,
R⁴ represents hydrogen or (C₁-C₆) alkyl,
X represents C(O)O-, C(O)NH- or (C₁-C₆) alkylene,
Y represents (C₁-C₄) alkylene-oxy,
Z represents (C₅-C₅₀) alkyl,
m represents 0 or 1,
Het represents an N-heterocycle having 5 or 6 ring members and 1 or 2 heteroatoms, at least one heteroatom being N and the other heteroatom being selected from N, O or S, and bonding to the C atom of the polymer backbone preferably taking place via the at least one N-heteroatom of the heterocycle, and at least one CO group preferably being contained in the heterocycle, which CO group is preferably arranged in the ortho position with respect to the at least one N-heteroatom, and it being possible for the heterocycle to optionally also carry additional substituents, in particular selected from C₁₋₆ alkyl and halogen, and it being possible for the hydrocarbon functional groups to optionally also be mono- or polysubstituted, in particular by functional groups selected from halogen functional groups, hydroxy functional groups, alkoxy functional groups, amino functional groups, alkylamino functional groups, dialkylamino functional groups, aryl functional groups, arylalkyl functional groups and cycloaliphatic functional groups, **characterized in that** the hydrophobically modified polysaccharide is inulin lauryl carbamate or cetyl hydroxyethylcellulose or mixtures thereof.

2. The O/W emulsion according to claim 1, **characterized in that** the hydrophobically modified polysaccharide is contained in an amount of 0.1-3.0 wt.%.

3. The O/W emulsion according to one of claims 1 or 2, **characterized in that** the fatty substance is contained in an amount of 5.0-30.0 wt.%.

4. The O/W emulsion according to one of claims 1 to 3, **characterized in that** a 1% aqueous solution of the hydrophobically modified polysaccharide has a surface tension of below 60 mN/m.

5. The O/W emulsion according to one of claims 1 to 4, **characterized in that** a 1% aqueous solution of the hydrophobically modified polysaccharide has an interfacial tension against the oil phase of below 25 mN/m.

6. The O/W emulsion according to one of claims 1 to 5, **characterized in that** the acrylamido-alkanoic acid is an acrylamido-sulfonic acid.

7. The O/W emulsion according to claim 6, **characterized in that** the acrylamido-alkanoic-acid-based copolymer is ammonium acryloyldimethyltaurate/VP copolymer.

8. The O/W emulsion according to one of claims 1 to 7, **characterized in that** the fatty substance is a polar or nonpolar liquid oil or mixtures thereof.

9. The O/W emulsion according to one of claims 1 to 8, **characterized in that**, at a temperature of 25 °C, said emulsion has a viscosity of from 500 to 10000 Pas at a shear rate of 0.1 s⁻¹ and a viscosity of from 1 to 10 Pas at a shear rate of 100 s⁻¹.

10. The O/W emulsion according to one claims 1 to 10, **characterized in that** said emulsion is an emulsifier-free preparation.

11. The use of an O/W emulsion according to one of claims 1 to 10 for treating facial skin.

12. The use of an O/W emulsion according to one of claims 1 to 10 for producing a composition for treating sensitive or dry skin.

13. The use of an O/W emulsion according to one of claims 1 to 10 for producing a composition for preventing sensitive or dry skin and/or for preventing skin reddening and skin irritation.

14. The use of an O/W emulsion according to one of claims 1 to 10 for treating mature skin.

15. The use of an O/W emulsion according to one of claims 1 to 10 for treating skin in sensitive regions, in particular in the region of the eyes.

16. The use of an O/W emulsion according to one of claims 1 to 10 for producing a composition for treating stressed, in particular UV-stressed, skin.

17. The use of an O/W emulsion according to one of claims 1 to 10 for producing a composition for treating the skin following a chemical or mechanical exfoliation treatment.

18. The use of an O/W emulsion according to one of claims 1 to 10 for producing a composition for transporting active ingredients into the skin.

19. A cosmetic or pharmaceutical composition, containing an O/W emulsion according to one of claims 1 to 10, **characterized in that** said composition is an eye cream or eye balm.

## Revendications

1. Émulsion H/E, contenant les constituants suivants :
a) au moins un polysaccharide hydrophobiquement modifié
b) 0,5 à 5,0 % en poids d'au moins un copolymère à base d'un acide acrylamidoalcanoïque,
c) au moins une matière grasse,
d) de l'eau,
dans laquelle le copolymère, selon b), est un copolymère à base d'un acide acrylamidoalcanoïque constitué de polymères comprenant des motifs de formule -CH₂-C(R¹)(W)- et des motifs de formule -CH₂-C(R²)([X]ₘ-[Y]ₙ-Z)- et/ou des motifs de formule - CH₂-C(R⁵)(Het)-,
R¹, R² et R⁵ représentant, indépendamment les uns des autres, l'hydrogène ou le méthyle, W représentant CONR³R⁴,
R³ représentant l'alkyle en (C₁-C₆)-CO₂H, en (C₁-C₆)-PO₃H ou en (C₁-C₆)-SO₃H,
R⁴ représentant l'hydrogène ou l'alkyle en (C₁-C₆),
X représentant des alkylènes C(O)O-, C(O)NH- ou en (C₁-C₆),
Y représentant des alkylènes-oxy en (C₁-C₄),
Z représentant l'alkyle en (C₅-C₅₀),
m représentant 0 ou 1,
Het représentant un N-hétérocycle à 5 ou 6 chaînons et à 1 ou 2 hétéroatomes, au moins 1 hétéroatome étant N et l'autre hétéroatome étant choisi parmi N, O ou S, et une liaison de préférence à l'atome C de l'épine dorsale du polymère s'effectuant par l'intermédiaire de l'au moins un N-hétéroatome de l'hétérocycle, et l'hétérocycle contenant de préférence au moins un groupe CO, qui est de préférence disposé en position ortho par rapport à l'au moins un N-hétéroatome, et l'hétérocycle pouvant éventuellement également porter d'autres substituants, en particulier choisis parmi l'alkyle en C₁₋₆ et l'halogène, et les radicaux hydrocarbonés pouvant éventuellement également être mono- ou polysubstitués, en particulier par des radicaux choisis parmi les radicaux halogènes, hydroxy, alcoxy, amino, alkylamino, dialkylamino, aryles, arylalkyles et cycloaliphatiques, **caractérisée en ce que** le polysaccharide hydrophobiquement modifié est de l'inuline lauryl carbamate ou de la cétyl hydroxyéthylcellulose ou leurs mélanges.

2. Émulsion H/E selon la revendication 1, **caractérisée en ce que** le polysaccharide hydrophobiquement modifié est contenu en une quantité de 0,1 à 3,0 % en poids.

3. Émulsion H/E selon l'une des revendications 1 ou 2, **caractérisée en ce que** la matière grasse est contenue en une quantité de 5,0 à 30,0 % en poids.

4. Émulsion H/E selon l'une des revendications 1 à 3, **caractérisée en ce qu'**une solution aqueuse à 1 % du polysaccharide hydrophobiquement modifié possède une tension superficielle inférieure à 60 mN/m.

5. Émulsion H/E selon l'une des revendications 1 à 4, **caractérisée en ce qu'**une solution aqueuse à 1 % du polysaccharide hydrophobiquement modifié possède une tension interfaciale contre la phase huileuse inférieure à 25 mN/m.

6. Émulsion H/E selon l'une des revendications 1 à 5, **caractérisée en ce que** l'acide acrylamidoalcanoïque est un acide acrylamidosulfonique.

7. Émulsion H/E selon la revendication 6, **caractérisée en ce que** le copolymère à base d'acide acrylamidoalcanoïque est un copolymère d'acryloyldiméthyltaurate d'ammonium/VP.

8. Émulsion H/E selon l'une des revendications 1 à 7, **caractérisée en ce que** la matière grasse est une huile liquide polaire ou non polaire ou leurs mélanges.

9. Émulsion H/E selon l'une des revendications 1 à 8, **caractérisée en ce qu'**elle présente, à une température de 25 °C, une viscosité de 500 à 10 000 Pas à une vitesse de cisaillement de 0,1 s⁻¹ et une viscosité de 1 à 10 Pas à une vitesse de cisaillement de 100 s⁻¹.

10. Émulsion H/E selon l'une des revendications 1 à 10, **caractérisée en ce qu'**il s'agit d'une préparation sans émulsifiant.

11. Utilisation d'une émulsion H/E selon l'une des revendications 1 à 10 pour le traitement de la peau du visage.

12. Utilisation d'une émulsion H/E selon l'une des revendications 1 à 10 pour la production d'une composition permettant le traitement des peaux sensibles ou sèches.

13. Utilisation d'une émulsion H/E selon l'une des revendications 1 à 10 pour la fabrication d'une composition contre les peaux sensibles ou sèches et/ou contre les rougeurs et les irritations cutanées.

14. Utilisation d'une émulsion H/E selon l'une des revendications 1 à 10 pour le traitement des peaux matures.

15. Utilisation d'une émulsion H/E selon l'une des revendications 1 à 10 pour le traitement des zones sensibles ou délicates de la peau, en particulier la zone des yeux.

16. Utilisation d'une émulsion H/E selon l'une des revendications 1 à 10 pour la fabrication d'une composition permettant le traitement des peaux stressées, en particulier des peaux exposées aux UV.

17. Utilisation d'une émulsion H/E selon l'une des revendications 1 à 10 pour la fabrication d'une composition de traitement de la peau après un traitement de peeling chimique ou mécanique.

18. Utilisation d'une émulsion H/E selon l'une des revendications 1 à 10 pour la fabrication d'une composition permettant le transport de principes actifs dans la peau.

19. Composition cosmétique ou pharmaceutique contenant une émulsion H/E selon l'une des revendications 1 à 10, **caractérisée en ce qu'**il s'agit d'une crème ou d'une pommade pour les yeux.
